# EUROPEAN PATENT APPLICATION

(11) **EP 2 671 583 A1**
(43) Date of publication of application: **11.12.2013**
(21) Application number: 12742647.6
(22) Date of filing: 30.01.2012
(51) Int. Cl.: A61K 31/436, A61K 9/06, A61K 47/10, A61K 47/18, A61K 47/32, A61P 17/00, A61P 17/02, A61P 17/08, A61P 35/00, A61P 37/08, C07D 498/18

(54) **EXTERNALLY-USED DRUG FOR TREATING SKIN DISORDER AND METHOD FOR PRODUCING SAME**

(30) Priority: 31.01.2011 JP 2011018273
(71) Applicant: Osaka University, Suita-shi, Osaka 565-0871 (JP)
(72) Inventor: KANEDA, Mari, Osaka 565-0871 (JP); KATAYAMA, Ichiro, Osaka 565-0871 (JP)
(74) Representative: Cabinet Plasseraud
(86) International application number: PCT/JP2012/052047
(87) International publication number: WO 2012/105521

(57) **Abstract**

An external medicine of the present invention includes a gel composition or an ointment composition, each of which contains sirolimus and/or a derivative thereof. Accordingly, provided is an external medicine which enables sirolimus, in an amount sufficient to treat a skin disease, to be absorbed into an affected part without causing a side effect.

## Description

### Technical Field

The present invention relates to (i) an external medicine for treating a skin disease and (ii) a method for producing the external medicine.

### Background Art

In recent years, an onset of a skin disease has been increasing in accordance with a change in living environment etc. In order to treat the skin disease, it is important to know a molecular mechanism of the onset of the skin disease. Studies on the molecular mechanism of the onset of the skin disease have been conducted with the use of biotechnology, and various causative genes of the skin disease have been identified so far.

For example, there has been known a genetic disease called tuberous sclerosis complex (TSC). This genetic disease causes (i) systemic tumors such as a skin tumor, (ii) central nervous system damage (e.g., epilepsy, delayed psychological development, and autism disorder), and (iii) vitiligo. It is known that causative genes of this genetic disease are (i) TSC1 which encodes protein hamartin and (ii) TSC2 which encodes protein tuberin. A complex of the protein hamartin and the protein tuberin be upstream of mTOR, and suppresses a function of mTOR. It is considered that tuberous sclerosis complex is developed in the following mechanism. That is, at least one of the protein hamartin and the protein tuberin becomes non-functional, and this causes mTOR to be unsuppressed. As a result, tuberous sclerosis complex is developed. Inhibition of mTOR is effective for treating tuberous sclerosis complex developed in such a mechanism. In view of this, use of sirolimus, which is an inhibitory agent of mTOR, has been tried for treating tuberous sclerosis complex.

In a foreign country, sirolimus has been systemically administered to a patient with tuberous sclerosis complex in an attempt to treat a brain tumor, a kidney tumor, and a lung tumor of the patient. It is reported that a good treatment result has been obtained. However, it has been found that, once the administration of sirolimus is stopped, the foregoing tumors increase again. Therefore, it is considered necessary to continue systemically administering sirolimus for a long period so as to keep the tumors reduced. This result applies not only to the brain tumor, the kidney tumor, and the lung tumor but also to the skin tumor. In order to treat this skin tumor, it is necessary to systemically administer sirolimus for a long period.

However, systemic administration of sirolimus has a disadvantage of causing a side effect. It is therefore difficult to continue administering sirolimus for a long period. In terms of this, it is considered to topically apply sirolimus to the skin tumor of tuberous sclerosis complex in order to treat the skin tumor without causing a side effect. Accordingly, development of an external medicine of sirolimus has been conducted (see Non-Patent Literatures 1 through 3). Non-Patent Literatures 1 and 2 describe applying the external medicine of sirolimus to angiofibroma formed on the face of a patient with tuberous sclerosis complex. Non-Patent Literature 3 describes applying an ointment containing sirolimus to a skin tumor of an animal model with tuberous sclerosis complex.

Further, it has been tried to treat a skin disease, other than the skin tumor, of tuberous sclerosis complex with the use of an external medicine of sirolimus (see Non-Patent Literatures 4 and 5). Non-Patent Literature 4 describes applying prescribed medication containing sirolimus to a psoriasis vulgaris of a human. Non-Patent Literature 5 describes topically applying sirolimus to a port-wine stain of a human.

Furthermore, it has been tried to administer, in various forms, a macrolide such as sirolimus to a patient (see Non-Patent Literatures 1 and 6).

### Citation List

### [Patent Literature]

Patent Literature 1
   Japanese Patent Application Publication, Tokuhyo, No. 2008-533153 A (Publication Date: August 21, 2008)

### [Non-Patent Literatures]

Non-Patent Literature 1
   Anna K. Haemel, et. al., Arch Dermatol. Jul;146(7):715-8, 2010
Non-Patent Literature 2
   Elizabeth Kaufman Mcnamara, et. al., Journal of Dermatological Treatment, Early Online, 1-3, 2010
Non-Patent Literature 3
   Aubrey Rauktys, et. al., BMC Dermatology, 8:1, 2008
Non-Patent Literature 4
   A. D. Ormerod, et. al., British Journal of Dermatology, 152, p.758-764, 2005
Non-Patent Literature 5
   Thuy L. Phung, et. al., Lasers in Surgery and Medicine 40:1-5, 2008
Non-Patent Literature 6
   Abstract of the 61th Annual Meeting of the Japanese Dermatological Association, September 11, 2010

### Summary of Invention

### Technical Problem

It is generally known that, in a case where a molecular weight of a compound used as an external medicine is great, the compound is less absorbed percutaneously because a molecular size of the compound increases, or diffusivity of the compound decreases (Otani Michiteru, Stock of Knowledge about Application (Nurigusuri no unchiku), VOL. 2, maruho, Maruho Co., Ltd., p.4, 2009). Therefore, the compound used as an external medicine is recommended to have a molecular weight of 500 Dalton or less.

However, the molecular weight of sirolimus is 914 Dalton. Therefore, sirolimus is poorly absorbed percutaneously. Non-Patent Literatures 1 and 2 describe that use of the external medicine of sirolimus improved the angiofibroma. However, the external medicines of sirolimus of Non-Patent Literatures 1 and 2 have not developed to a practical level. Development of an external medicine which enables sirolimus to be more absorbed percutaneously is desired.

Non-Patent Literature 3 describes that a sirolimus concentration in blood, measured 24 hours after application of an ointment containing 0.4% by mass of sirolimus to the skin tumor, was 6.3 ± 0.6 ng/mL. Similarly, Non-Patent Literature 3 describes that a sirolimus concentration in blood, measured 24 hours after application of an ointment containing 0.8% by mass of sirolimus to the skin tumor, was 12.3 ± 1.5 ng/mL. Such sirolimus concentrations in blood are almost identical to a general sirolimus concentration in blood (5 to 10 ng/mL) at systemic administration of sirolimus. As just described, the technique of Non-Patent Literature 3 has an effect similar to systemic administration of sirolimus because sirolimus moves into blood even though sirolimus is topically applied. As a result, a side effect occurs.

Further, according to Non-Patent Literature 3, a sirolimus concentration in a skin tumor, measured 24 hours after application of an ointment containing 0.4% by mass of sirolimus to the skin tumor, was 13.8 ± 0.7 ng/mL. This concentration is approximately 1.9 times to 2.5 times higher than the sirolimus concentration in blood (6.3 ± 0.6 ng/mL). A sirolimus concentration in a skin tumor, measured 24 hours after application of an ointment containing 0.8% by mass of sirolimus to the skin tumor, was 59.6 ± 23.5 ng/mL. This concentration is approximately 2.6 times to 7.7 times higher than the sirolimus concentration in blood (12.3 ± 1.5 ng/mL). As just described, in a case where the ointment disclosed in Non-Patent Literature 3 is applied, a concentration of sirolimus that is present in the skin tumor is only several times higher than a concentration of sirolimus that moves into blood. In terms of this, the ointment disclosed in Non-Patent Literature 3 is not suitable for topical application.

The prescribed medication disclosed in Non-Patent Literature 4 is a lotion including sirolimus, capric acid, isopropyl myristate, and benzyl alcohol. A content of sirolimus in the prescribed medication is 2.2% by mass or 8% by mass. By applying the prescribed medication to an affected part, a side effect such as contact dermatitis and irritating sensation is caused. Further, it is reported that there was a patient whose affected part was not improved even though the prescribed medication containing 2.2% by mass of sirolimus had been applied to the affected part. Furthermore, the sirolimus concentration of 8% by mass is a concentration that is beyond technical common knowledge of a person skilled in the art. It is not practical to use a gel of such a concentration as an external medicine. Further, according to Non-Patent Literature 5, topical application of sirolimus to an affected part is not enough to treat a port-wine stain. Therefore, it is necessary to irradiate an affected part with a laser beam, in addition to applying sirolimus. As just described, according to the techniques of Non-Patent Literatures 4 and 5, sirolimus is poorly absorbable percutaneously, and it is therefore necessary to perform the sirolimus treatment by applying a large amount of sirolimus to an affected part or in combination with another treatment.

According to Non-Patent Literature 1, no study is conducted as to whether or not sirolimus actually brings about a therapeutic effect with respect to various types of diseases.

The present invention has been made in view of the above problems, and an object of the present invention is to provide an external medicine which enables sirolimus, in an amount sufficient to treat a skin disease, to be absorbed into an affected part without causing a side effect.

### Solution to Problem

In order to address the problem, the inventors made diligent study. As a result, the inventors found that by applying, to skin, (i) a product obtained by gelating a solution containing sirolimus and/or a derivative thereof (active ingredients) or (ii) a product obtained by dissolving at least one of the active ingredients in a solvent which was not compatible with the active ingredients, it was possible to improve significantly absorption of sirolimus into an affected part through skin as compared with a case in which a conventional external medicine containing sirolimus was applied to skin. Thus, the inventors have achieved the present invention based on the finding.

That is, an external medicine, of the present invention, for topically treating a skin disease includes a gel composition or an ointment composition, each of which contains sirolimus and/ or a derivative thereof. As used herein, the term "topically treating" indicates that the external medicine topically applied causes no systemic effect. Application of the external medicine, which has the above feature, to skin not only cause a significant improvement in absorption of sirolimus and a derivative thereof into an affected part through skin, but also causes sirolimus and the derivative thereof to stay in the affected part without leaking into blood. That is, with the use of the external medicine of the present invention, it is possible to treat a skin disease effectively and, in addition, there is exhibited any systemic effect caused by sirolimus and the derivative thereof (no side effect is produced).

### Advantageous Effects of Invention

When percutaneously applied, an external medicine of the present invention exhibits an effect that sirolimus and/or a derivative thereof can be absorbed, in an amount sufficient to treat a skin disease, into an affected part without causing a side effect.

### Brief Description of Drawings

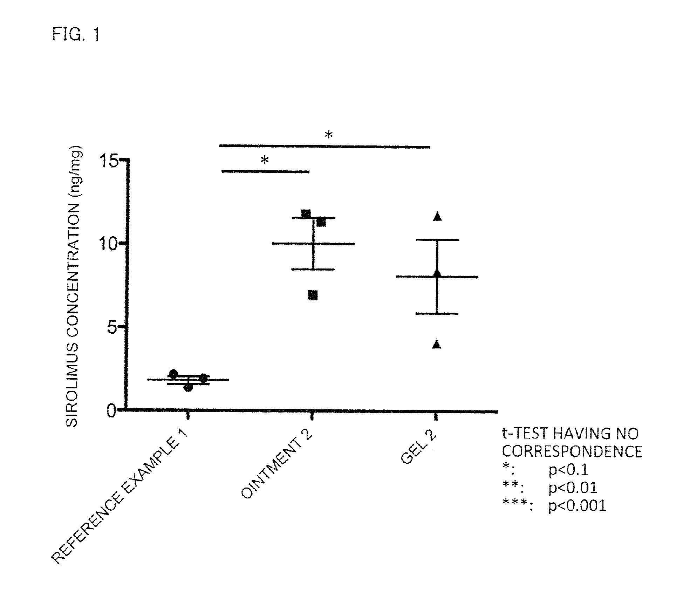

Fig. 1 is a view showing results obtained with use of various external medicines.

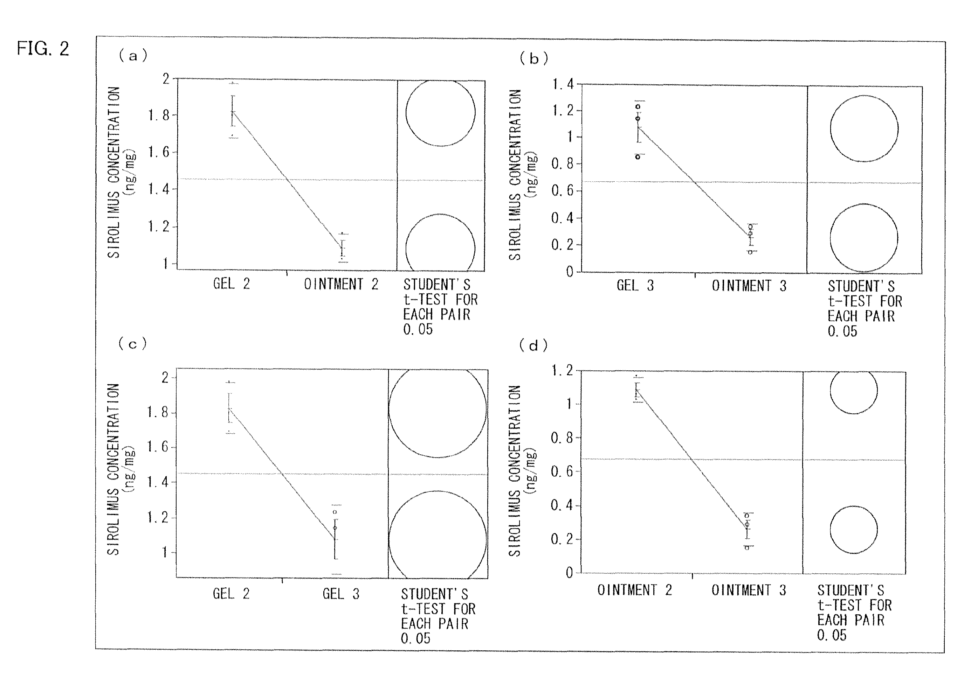

(a) through (d) of Fig. 2 are views showing results obtained with use of various external medicines.

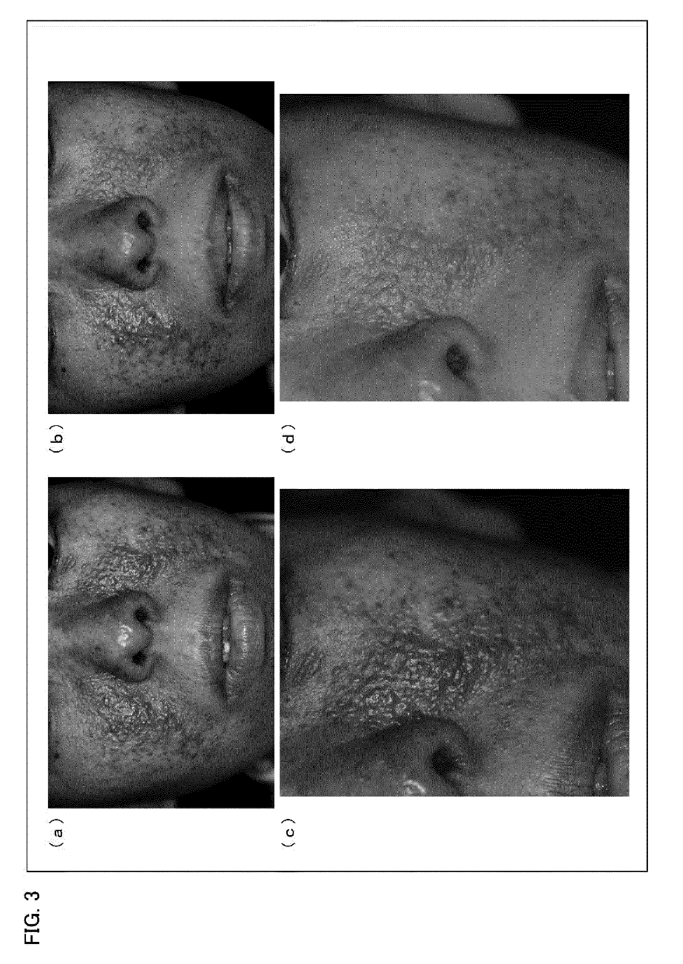

(a) of Fig. 3 is a view showing angiofibroma in a state before an external medicine was applied. (b) of Fig. 3 is a view showing angiofibroma in a state after the external medicine was applied. (c) of Fig. 3 is an enlarged view of a part of (a) of Fig. 3. (d) of Fig. 3 is an enlarged view of a part of (b) of Fig. 3.

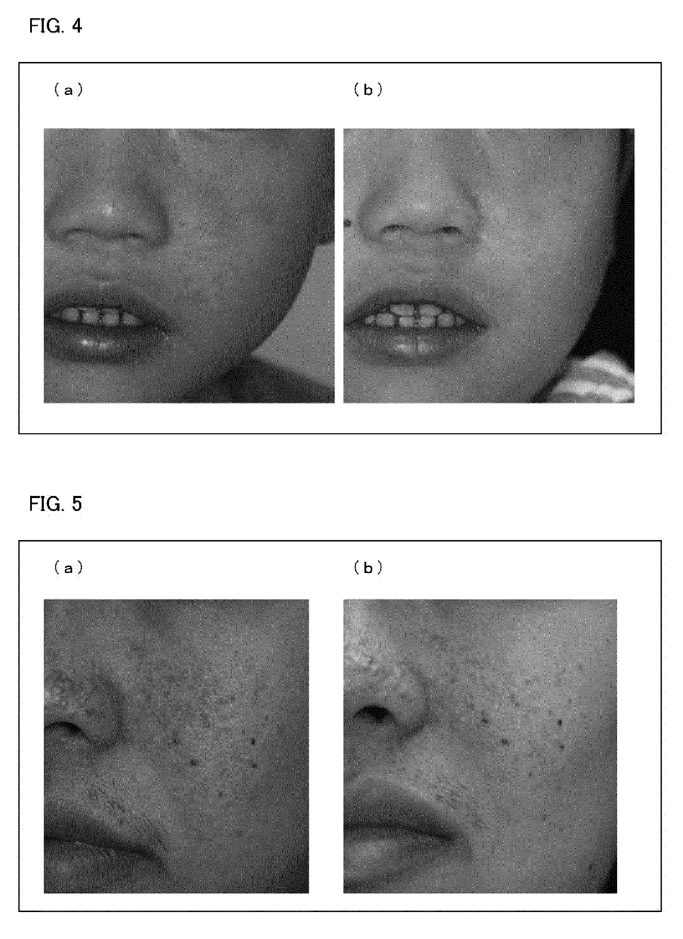

(a) of Fig. 4 is a view showing angiofibroma in a state before an external medicine was applied. (b) of Fig. 4 is a view showing angiofibroma in a state after the external medicine was applied.

(a) of Fig. 5 is a view showing angiofibroma in a state before an external medicine was applied. (b) of Fig. 5 is a view showing angiofibroma in a state after the external medicine was applied.

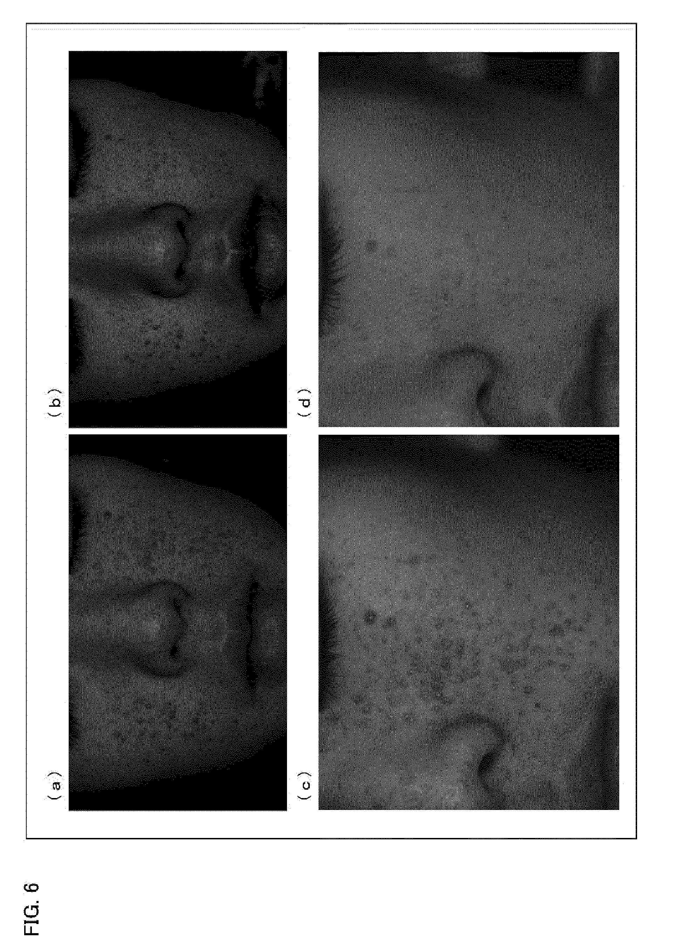

(a) of Fig. 6 is a view showing angiofibroma in a state before an external medicine was applied. (b) of Fig. 6 is a view showing angiofibroma in a state after the external medicine was applied. (c) of Fig. 6 is an enlarged view of a part of (a) of Fig. 6. (d) of Fig. 6 is an enlarged view of a part of (b) of Fig. 6.

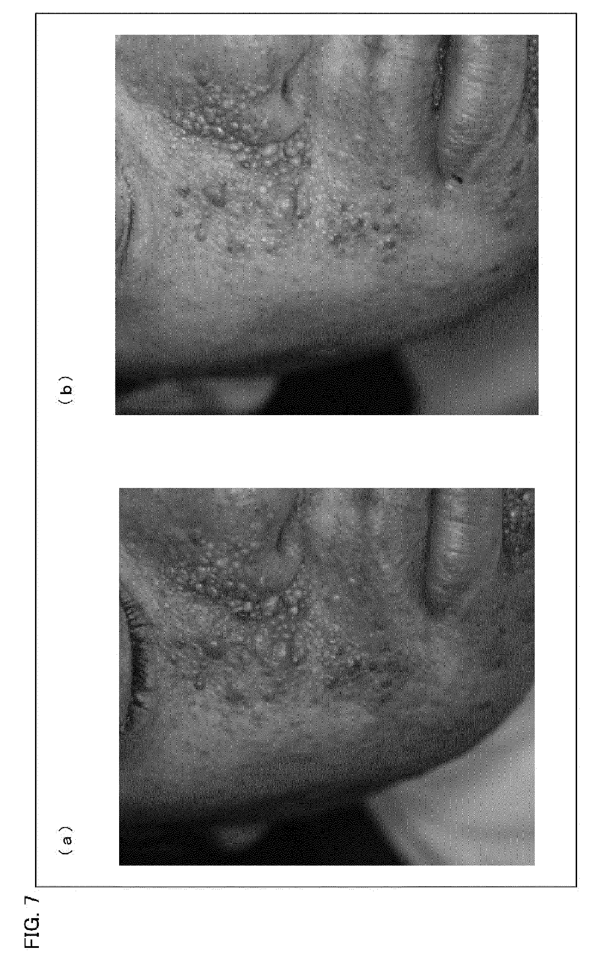

(a) of Fig. 7 is a view showing angiofibroma in a state before an external medicine was applied. (b) of Fig. 7 is a view showing angiofibroma in a state after the external medicine was applied.

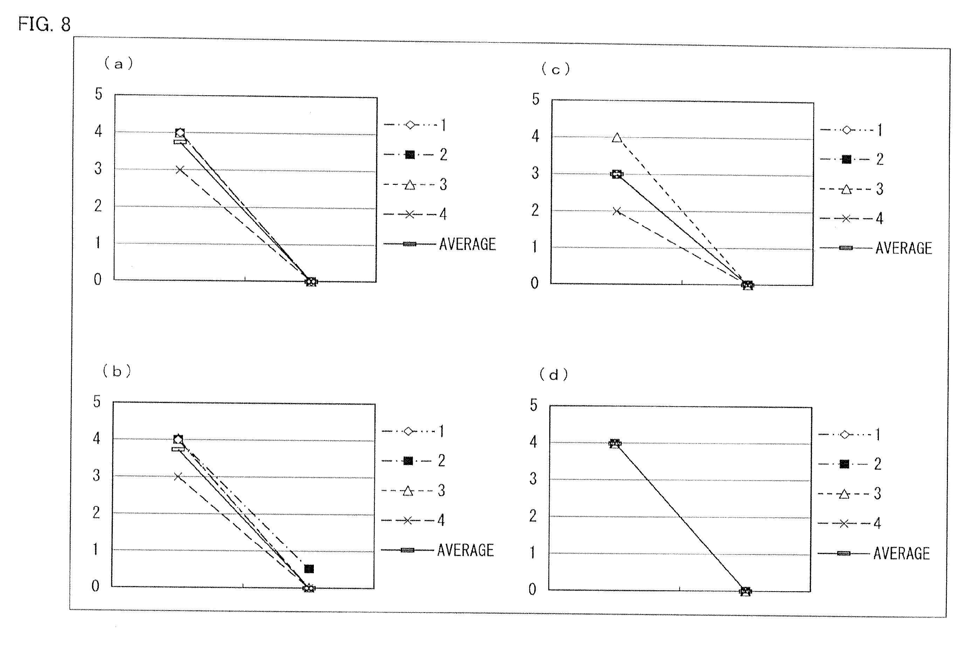

(a) through (d) of Fig. 8 are graphs showing results obtained with use of external medicines.

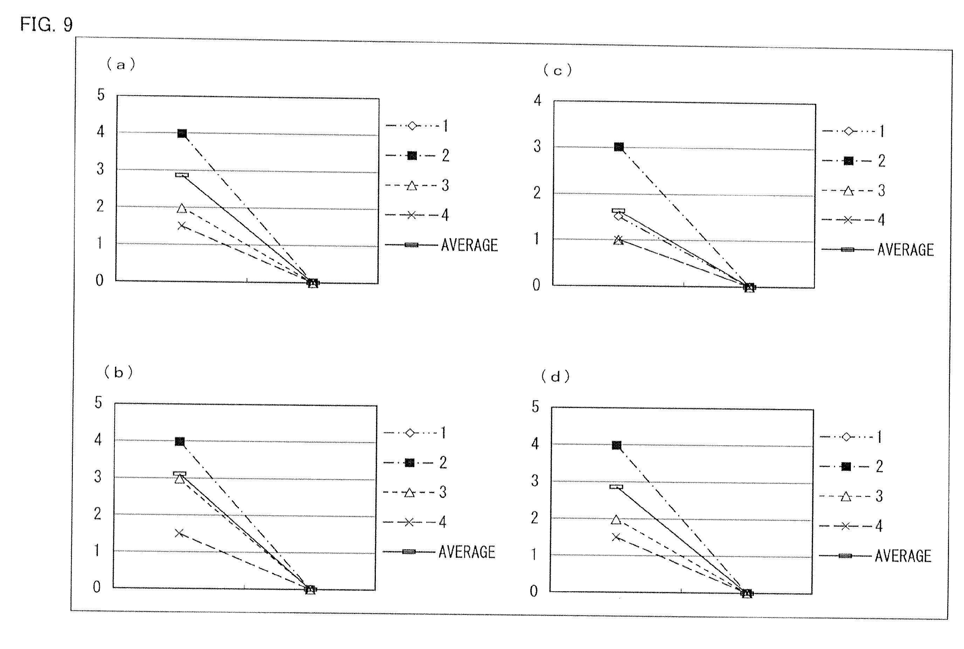

(a) through (d) of Fig. 9 are graphs showing results obtained with use of external medicines.

Fig. 10 is a graph showing a relative expression level of mRNA of MITF.

Fig. 11 is a graph showing a relative expression level of mRNA of TYR.

Fig. 12 is a graph showing a relative expression level of mRNA of TYRP.

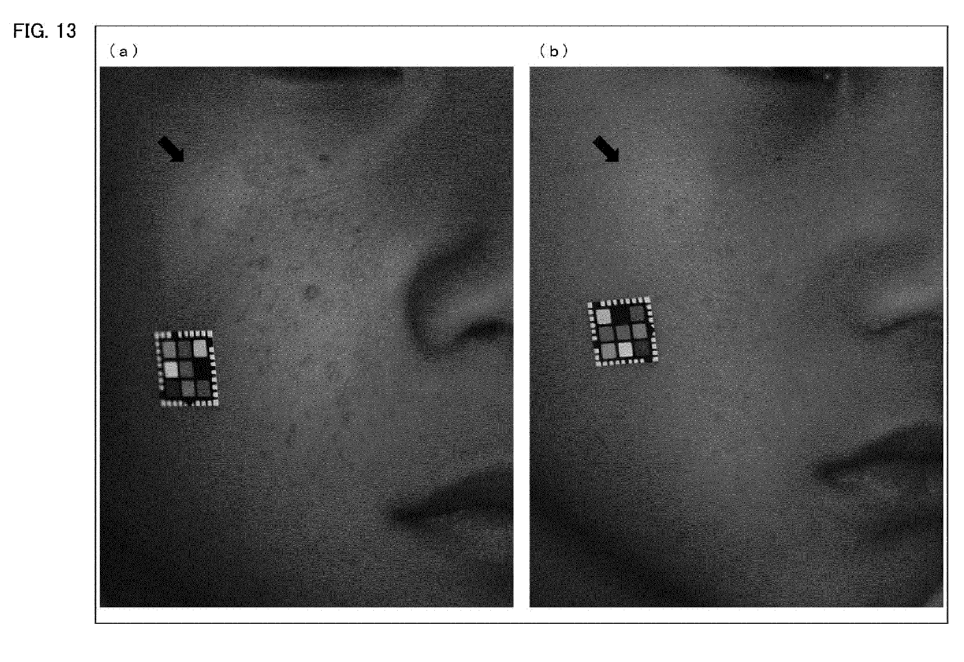

(a) of Fig. 13 is a view showing vitiligo in a state before an external medicine was applied. (b) of Fig. 13 is a view showing vitiligo in a state after the external medicine was applied.

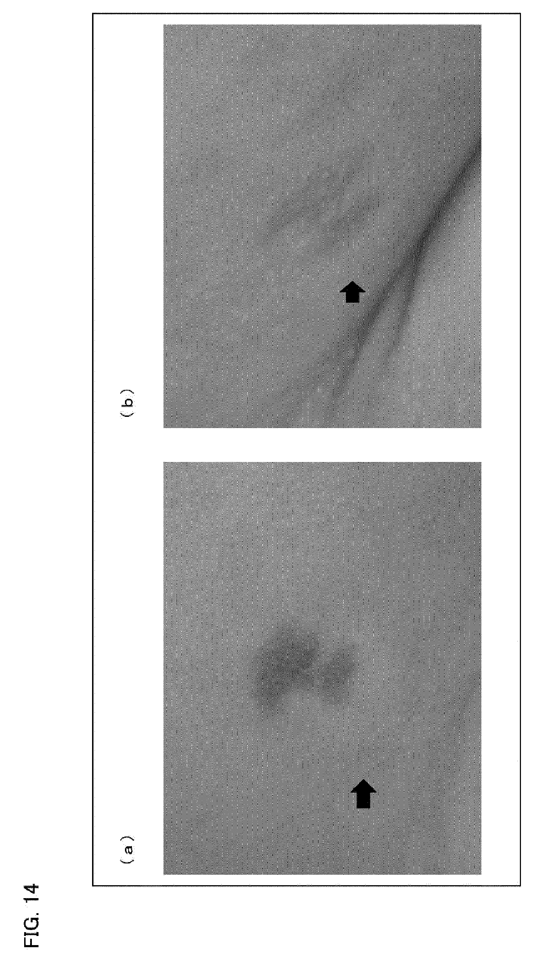

(a) of Fig. 14 is a view showing vitiligo in a state before an external medicine was applied. (b) of Fig. 14 is a view showing vitiligo in a state after the external medicine was applied.

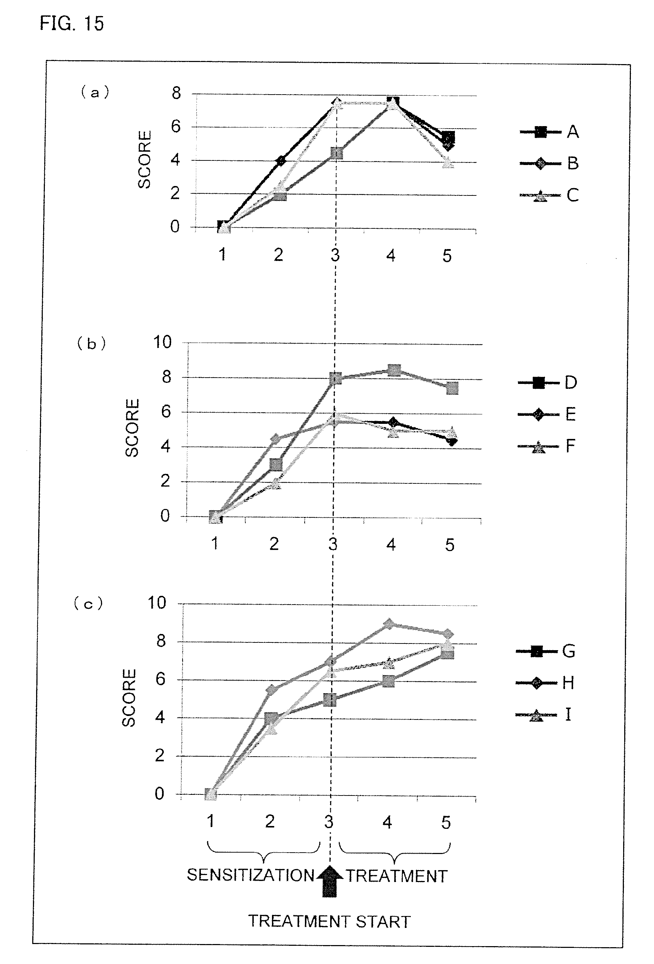

(a) through (d) of Fig. 15 are graphs showing results obtained with use of external medicines.

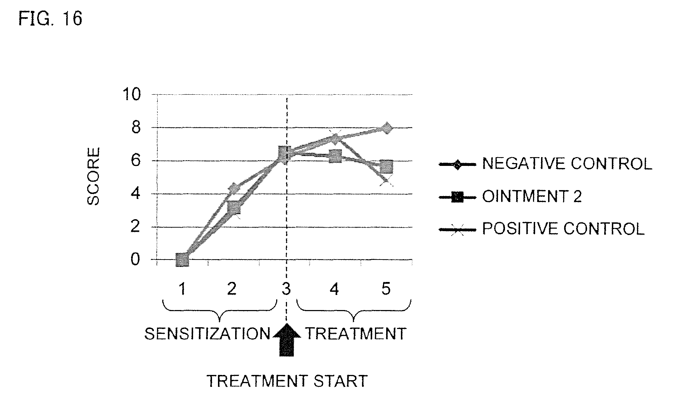

Fig. 16 is a graph showing results obtained with use of external medicines.

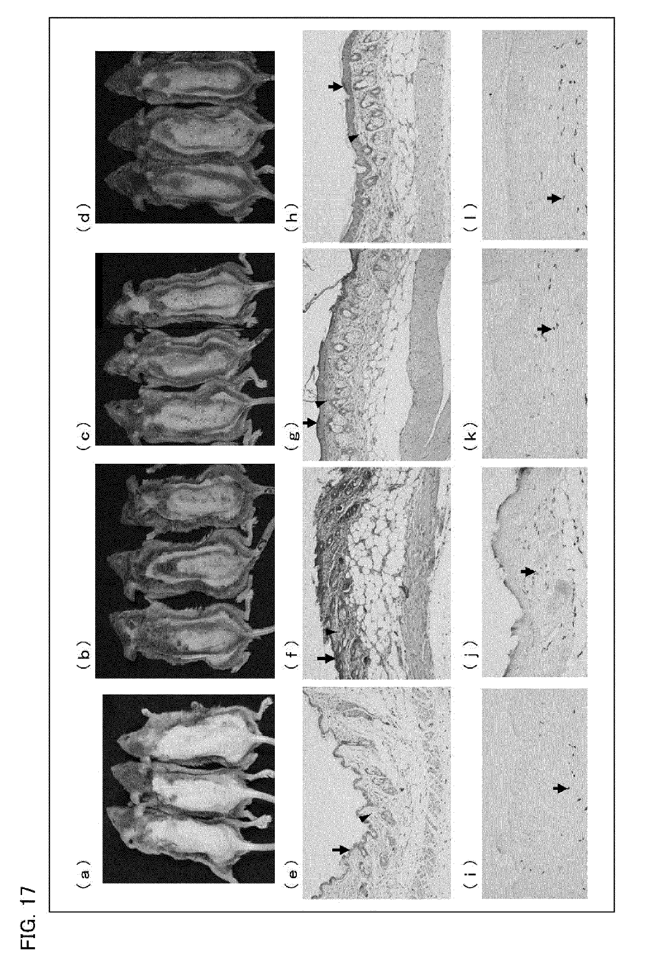

(a) through (d) of Fig. 17 are views of backs of mice. (e) through (h) of Fig. 17 are views of slices of mouse skin stained with hematoxylin and eosin. (i) through (1) of Fig. 17 are views of slices of mouse skin stained with toluidine blue.

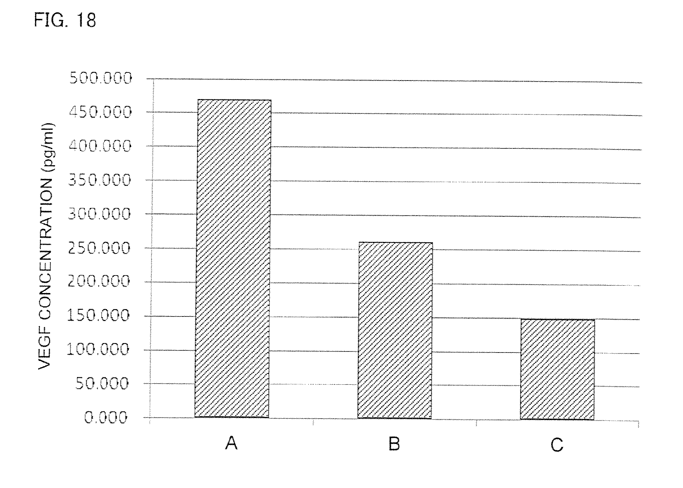

Fig. 18 is a view showing concentrations of VEGF expressed in a cell of severe angiofibroma, a cell of moderate angiofibroma, or a cell of mild angiofibroma, respectively.

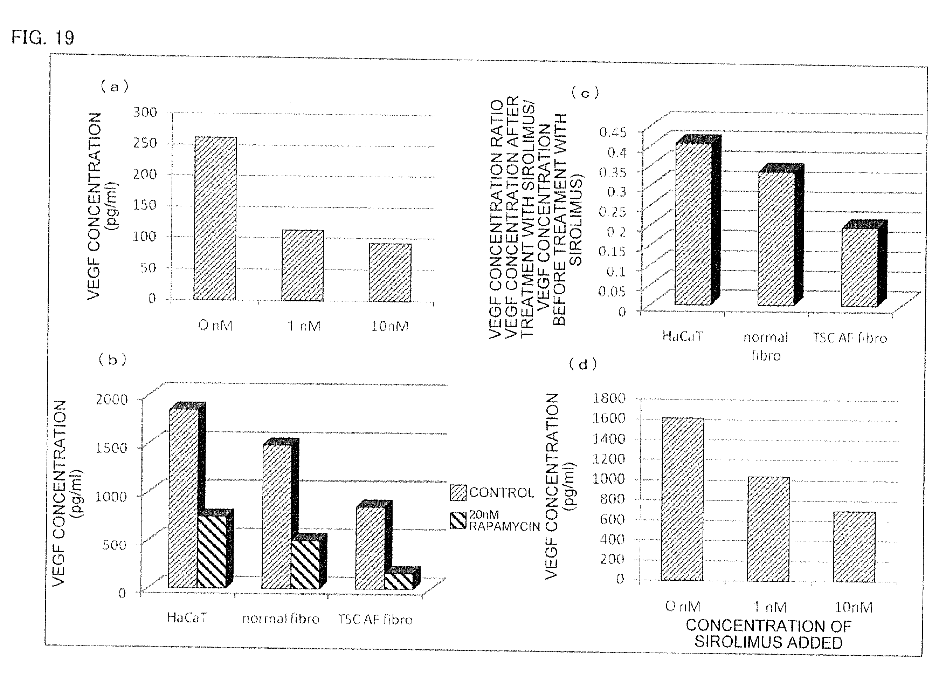

(a) of Fig. 19 is a graph showing concentrations (pg/mL) of VEGF expressed in respective cells of angiofibroma, which cells had been treated with 0 nM of sirolimus (untreated), 1 nM of sirolimus, and 10 nM of sirolimus, respectively. (b) of Fig. 19 is a graph showing concentrations (pg/mL) of VEGF expressed in a cell of angiofibroma (TSC AF fibro), a human fibroblast (normal fibro), and HaCat, respectively, before and after the cells were treated with sirolimus. (c) of Fig. 19 is a graph showing a rate of decrease (ratio: a concentration of VEGF after treatment with 20 nM of sirolimus / a concentration of VEGF before the treatment with 20 nM of sirolimus) in concentration of VEGF, which decrease was caused by treating each of the three types of cells with sirolimus. (d) of Fig. 19 is a graph showing concentrations (pg/mL) of VEGF expressed in respective cells treated with 0 nM of sirolimus (untreated), 1 nM of sirolimus, and 10 nM of sirolimus, respectively.

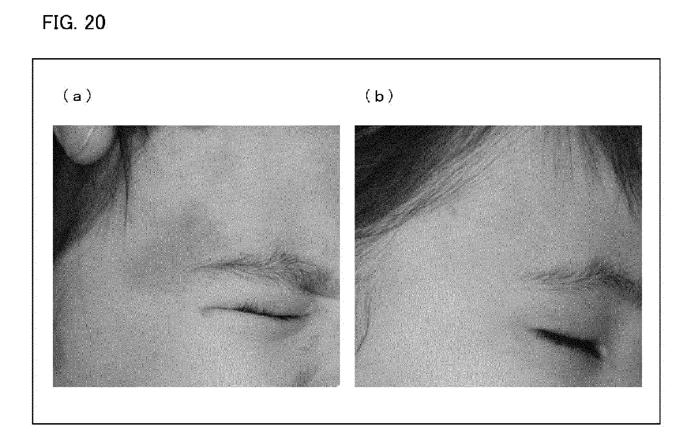

(a) of Fig. 20 is a view showing erythema in a state before an external medicine of the present invention was applied. (b) of Fig. 20 is a view showing erythema in a state after an external medicine of the present invention was applied.

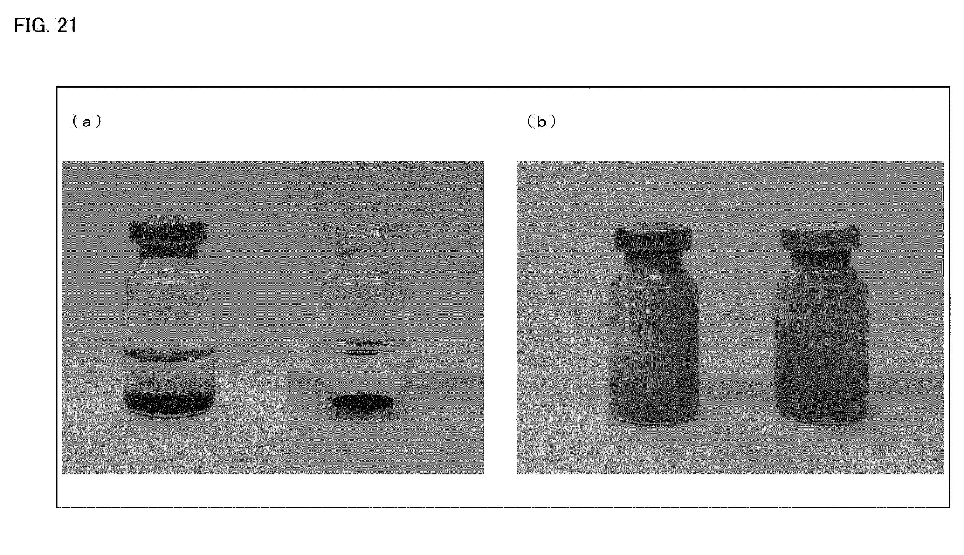

(a) of Fig. 21 is a view showing a state in which propylene carbonate was added to a component of an ointment prepared in the present example or to white petrolatum. (b) of Fig. 21 is a view showing a state in which a component of the ointment to which propylene carbonate had been added was stirred, or white petrolatum to which propylene carbonate had been added was stirred.

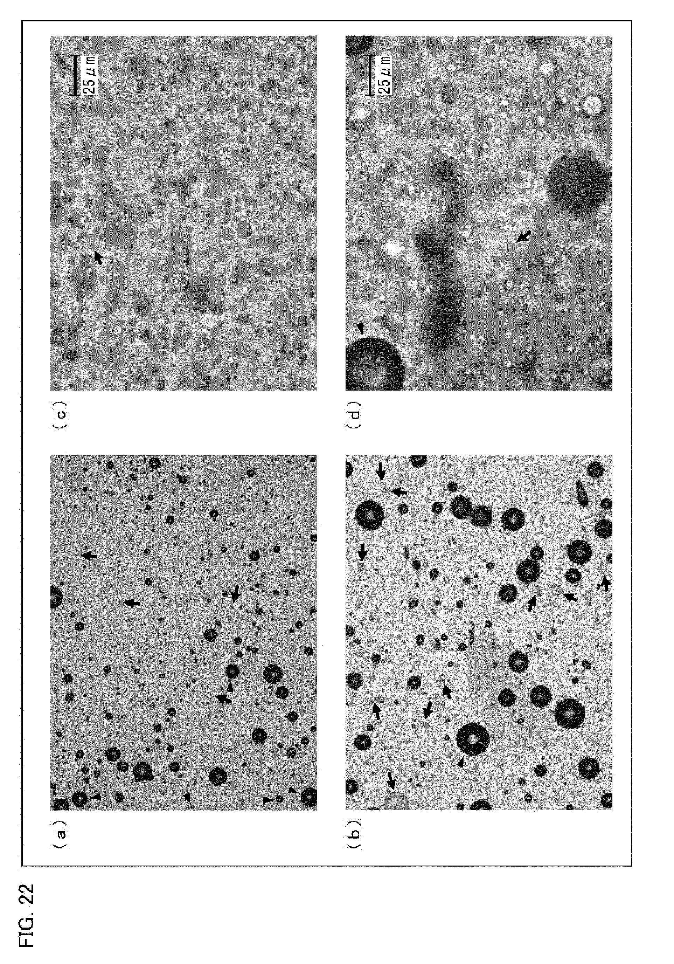

(a) of Fig. 22 is a view obtained by microscopically observing an ointment prepared with use of a component of an ointment prepared in the present example. (c) of Fig. 22 is an enlarged view of (a) of Fig. 22. (b) of Fig. 22 is a view obtained by observing, with use of a microscope, an ointment prepared with use of white petrolatum. (d) of Fig. 22 is an enlarged view of (b) of Fig. 22.

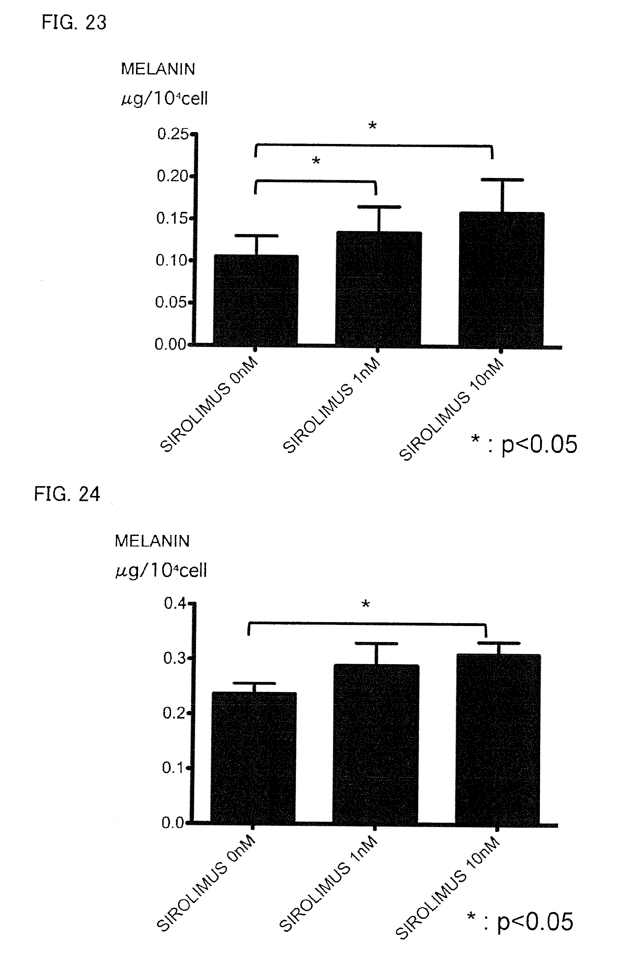

Fig. 23 is a graph showing an amount of melanin produced by melanocytes.

Fig. 24 is a graph showing an amount of melanin produced by melanocytes.

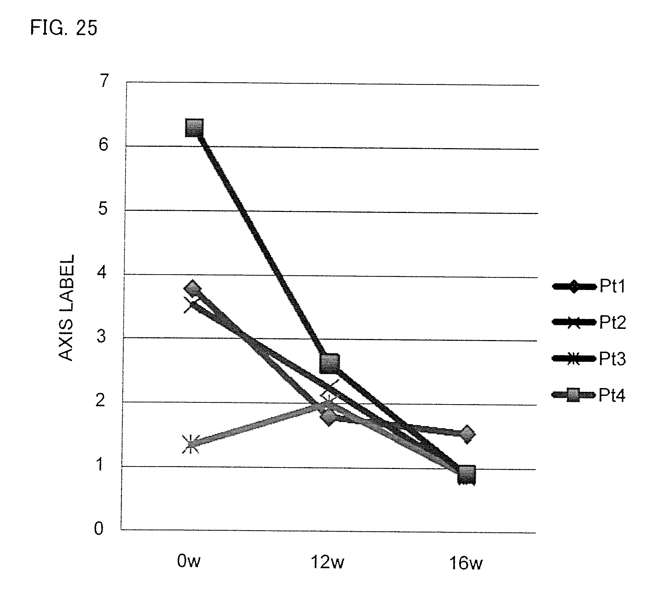

Fig. 25 is a graph showing how symptoms of four patients, each having vitiligo in a forehead region (a part which was exposed to light), underwent a change.

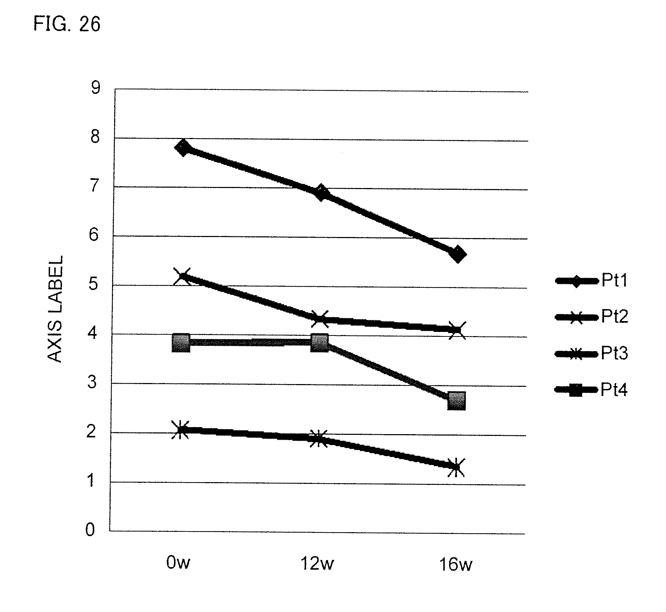

Fig. 26 is a graph showing how symptoms of four patients, each having vitiligo in an abdominal region (a part which was not exposed to light), underwent a change.

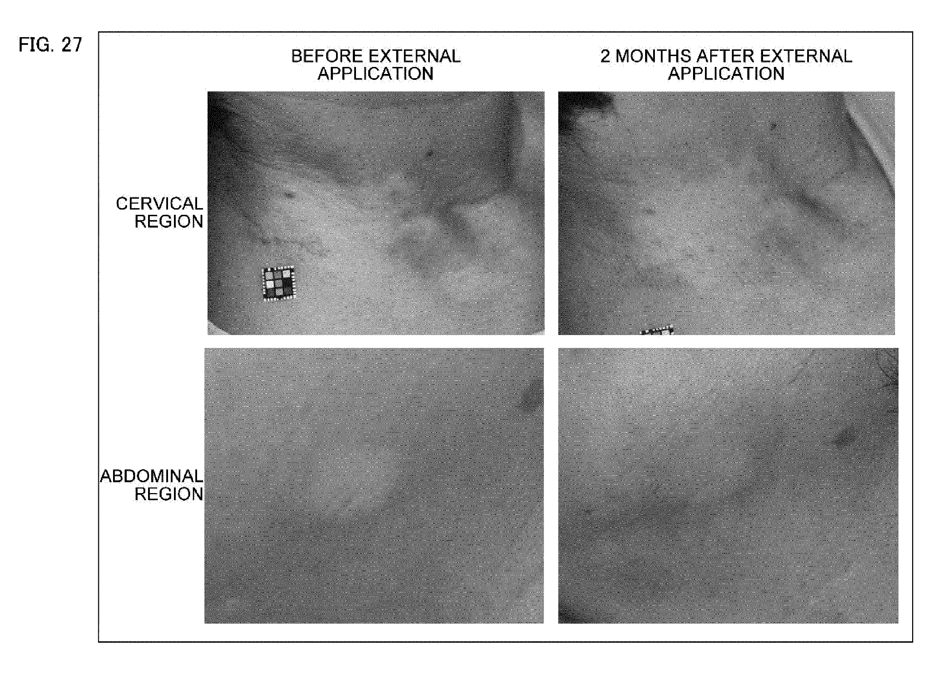

Fig. 27 is a view showing how a symptom of vitiligo vulgaris underwent a change.

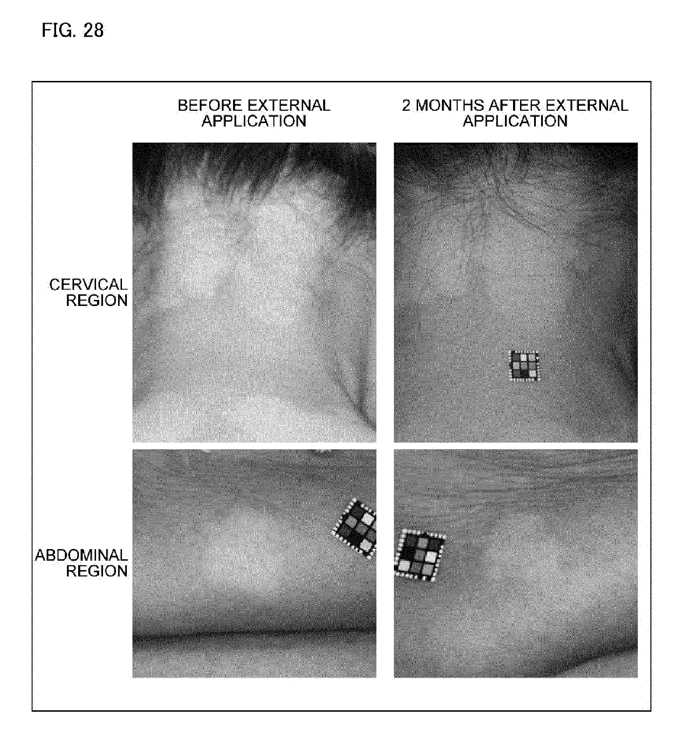

Fig. 28 is a view showing how a symptom of vitiligo vulgaris underwent a change.

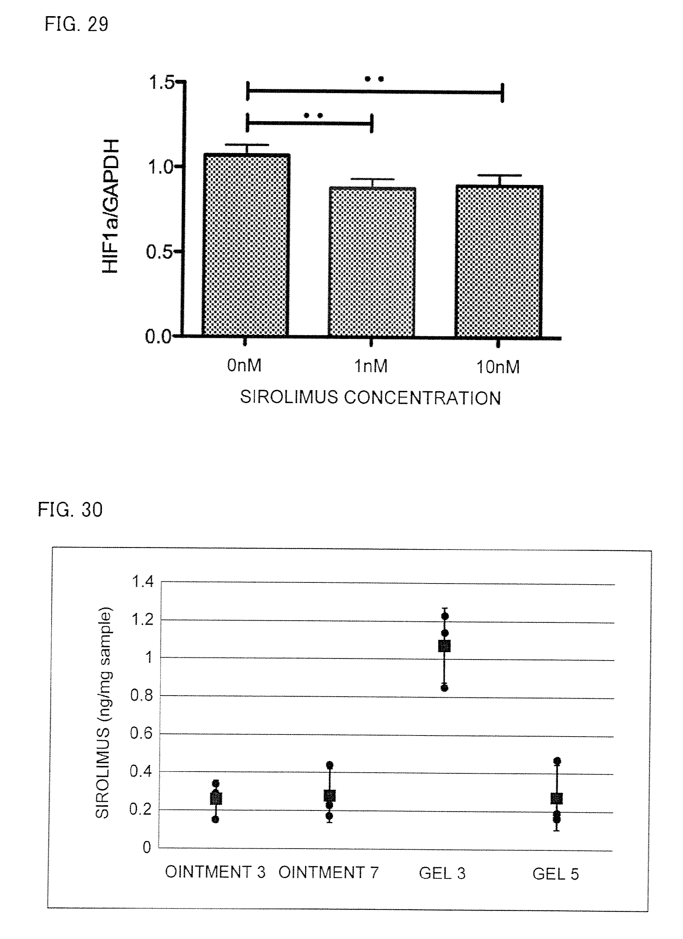

Fig. 29 is a graph showing an expression level of HIF1α in melanocytes.

Fig. 30 is a graph showing how a composition of a base affects an amount of an external medicine of the present invention absorbed via artificial skin.

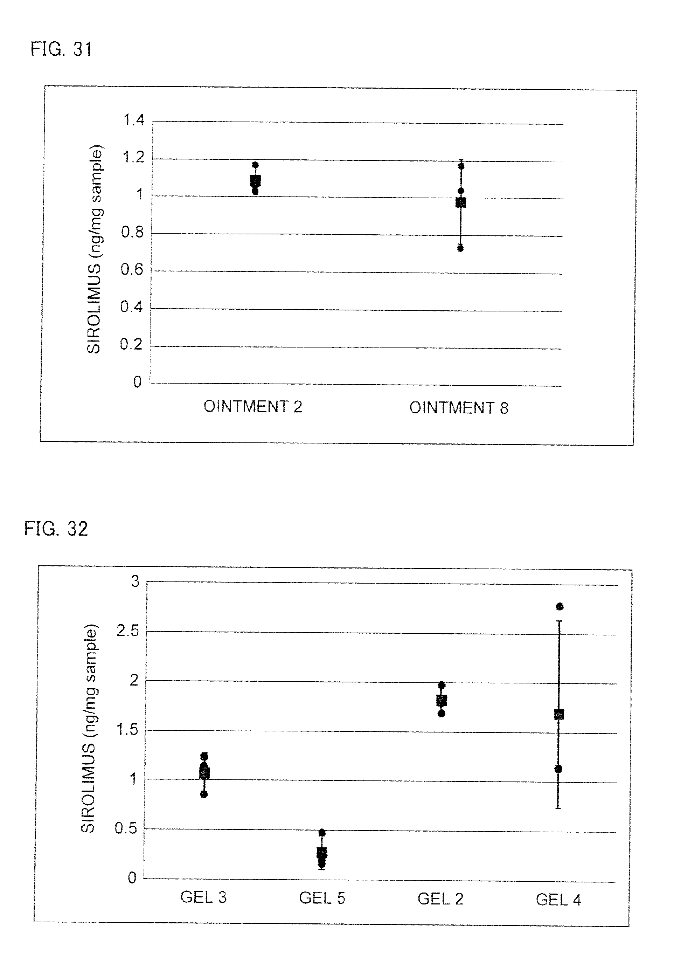

Fig. 31 is a graph showing how a composition of a base affects an amount of an external medicine of the present invention absorbed via artificial skin.

Fig. 32 is a graph showing how a composition of a base affects an amount of an external medicine of the present invention absorbed via artificial skin.

### Description of Embodiments

The following description will specifically discuss embodiments of the present invention.

### [Embodiment 1: External medicine including a gel composition]

The present invention provides an external medicine for topically treating a skin disease. As used herein, the term "treatment" indicates a reduction or an elimination of a symptom, and includes not only treatment that can be given for cure (after an onset of the skin disease) but also treatment that can be given for prevention (before an onset of the skin disease).

The external medicine of Embodiment 1 includes a gel composition which is obtained by gelating a solution containing an active ingredient for topically treating the skin disease. As used herein, the term "gel composition" indicates a jellylike solution in which an active ingredient is dissolved in a solvent which dissolves the active ingredient.

It is only necessary that the active ingredient be sirolimus and/or its derivative. The derivative of sirolimus is not limited to any particular one. Examples of the derivative include 42-O-(2-hydroxyethyl)sirolimus (everolimus, RAD001) and sirolimus42-[3-hydroxy-2-(hydroxymethyl)-2-methylpropanoate (temsirolimus).

It is known that such sirolimus and its derivative bring about the following effects by inhibiting mTOR: (i) inhibiting protein translation and synthesis, stopping a cell cycle, and causing apoptosis, so as to suppress a tumor and angiogenesis; (ii) inhibiting activation and proliferation of a T cell so as to suppress immunity by blocking a signaling pathway in which an IL2 receptor is involved; and (iii) inhibiting mast cell-mediated inflammation. Therefore, sirolimus and everolimus are each taken orally as an immunosuppressive agent which is used after a kidney transplant or a heart transplant. Further, everolimus is allowed in Japan to be used as an internal agent for a kidney malignancy. Temsirolimus is developed as an internal agent for a terminal liver cancer.

As described above, sirolimus and its derivative are mainly used as an internal medicine. Meanwhile, as described earlier, an external medicine of sirolimus is developed. However, an external medicine of sirolimus of a practical level has not been obtained because sirolimus is poorly absorbed percutaneously or causes a side effect by systemically demonstrating an effect even though sirolimus is topically applied. On the other hand, according to the present invention, the solution containing the active ingredient (sirolimus and its derivative) is gelated. This enables an increase in absorption efficiency of the active ingredient into an affected part through skin. As a result, it is possible to topically treat the skin disease with the use of the active ingredient.

As just described, in order to treat the skin disease topically, it is only necessary that the solution containing the active ingredient be gelated. In order to gelate the solution, a gelling agent can be used to gelate the solution. For example, in a case where Carbopol® 934NF is used as the gelling agent, it is possible to induce gelation of the solution by (i) adding Carbopol® 934NF in the solution and (ii) adjusting a pH of the solution to be neutral with the use of a pH adjuster such as tris(hydroxymethyl)aminomethane.

The external medicine of Embodiment 1 can be one that is made up of the gel composition. Alternatively, the external medicine of Embodiment 1 can further include another component (described later), provided that the gel composition maintains its gel form. Similarly, the gel composition can be one that is made up of the solution containing the active ingredient and a component which induces gelation (e.g., a gelling agent). The gel composition can further include another component, provided that the gel composition maintains its gel form. As just described, the gel composition of Embodiment 1 is one that is obtained by gelating the solution containing the active ingredient. The present invention also encompasses a composition in which the active ingredient is dissolved in the solvent which dissolves the active ingredient (the solution in which the active ingredient is dissolved), the composition being in a state where gelation of the solution has not been induced yet. Similarly, the present invention also encompasses a kit in which the active ingredient, the solvent which dissolves the active ingredient, and the component which induces gelation are separately included. The composition and the kit like above can be used to prepare the gel composition (and the external medicine of Embodiment 1). As a matter of course, the composition for preparing the gel composition can further include another component. The kit for preparing the gel composition can further include another component.

As used herein, the term "kit" indicates a package including a container(s) (e.g., a bottle, a plate, a tube, and a dish) which contain(s) a specific material. The kit preferably includes a manual for use of the material. As used herein in an aspect of the kit, the term "include (including)" indicates a state in which a material and/or the like is contained in any of containers constituting the kit. The kit of the present invention can be a package in which a plurality of different compositions are packed together. Furthermore, the kit of the present invention can be a package in which a container containing a composition in a solution state is packed. The kit of the present invention can include two or more different substances contained in a single container in a mixed state, or can include two or more different substances contained in respective different containers. The "manual" can be written or printed on a sheet of paper or on other medium. Alternatively, the "manual" can be written in an electronic medium such as a magnetic tape, a computer-readable disk or tape, and a CD-ROM. The kit of the present invention can be employed so as to compose the foregoing composition. Further, the kit can separately include substances contained in the foregoing composition, or can separately include the foregoing composition and the another component.

The foregoing solvent which dissolves the active ingredient is not limited in particular. Examples of the solvent include isopropanol, ethanol, and propylene carbonate. In a case of the gel composition, at least one of isopropanol and ethanol is preferable.

According to the present invention, the active ingredient is dissolved in the solvent which dissolves the active ingredient, and the resulting solution is gelated. This remarkably increases absorption of the active ingredient into an affected part through skin. With the use of the present invention, treatment of the skin disease is realized with a much smaller amount of sirolimus than an amount of sirolimus used in a conventional external medicine. Further, according to the present invention, since the solution containing the active ingredient is gelated, the active ingredient is likely to remain in an affected part without leaking into blood. With the use of the present invention, it is possible to treat the skin disease without causing a side effect, unlike the conventional external medicine described in the "Background Art".

An amount of the active ingredient contained in the external medicine of Embodiment 1 is preferably 0.01% by mass to 2% by mass, more preferably 0.03% by mass to 1% by mass, relative to the total mass of the external medicine. In a case where the amount of the active ingredient is within this range, it is possible to treat the skin disease without causing a side effect. Note that an amount of the active ingredient contained in the composition for preparing the external medicine is similar to the amount of the active ingredient contained in the external medicine. An amount of the active ingredient included in the kit for preparing the external medicine can be any amount, as long as the amount of the active ingredient ultimately contained in the external medicine produced falls within the range. Further, an amount of the active ingredient contained in the gel composition can be determined as appropriate by a person skilled in the art, provided that the amount of the active ingredient contained in the external medicine falls within the range.

An amount of the solvent which dissolves the active ingredient and which is contained in the external medicine (or the gel composition) of Embodiment 1 is not limited in particular. It is only necessary that the amount of the solvent is enough to dissolve the active ingredient sufficiently. For example, the amount of the solvent to be used is 100 to 300 times larger, preferably 120 to 250 times larger, more preferably 121.5 to 244 times larger, than the amount of the active ingredient. Note that an amount of the solvent which dissolves the active ingredient and which is contained in the composition for preparing the external medicine is similar to the amount of the solvent which dissolves the active ingredient and which is contained in the external medicine. An amount of the solvent which dissolves the active ingredient and which is included in the kit for preparing the external medicine is optional, provided that the amount of the solvent is enough to dissolve the active ingredient sufficiently.

An amount of the component which induces gelation and which is contained in the external medicine (or the gel composition) of Embodiment 1 is not limited in particular. It is only necessary that the amount of the component be enough to gelate the solution containing the active ingredient (i.e., to convert the external medicine or the composition into a gel form). For example, in a case of using, as the component which induces gelation, (i) a gelling agent such as Carbopol® and (ii) a pH adjuster for inducing gelation (e.g., tris(hydroxymethyl)aminomethane), an amount of the gelling agent is, for example, 1.6% by mass relative to the total mass of the external medicine, and an amount of the pH adjuster is, for example, 0.4% by mass relative to the total mass of the external medicine. Note that an amount of the component which induces gelation and which is included in the kit for preparing the external medicine can be any amount, provided that the amount of the component is sufficient for the produced external medicine to be in a gel from.

In Example (described later), the external medicine of Embodiment 1 is used to treat a skin tumor, vitiligo, and erythema which are caused by tuberous sclerosis complex. Further, an external medicine in an ointment dosage form (described in Embodiment 2) is used to treat a skin tumor, vitiligo, and erythema which are caused by tuberous sclerosis complex and to treat atopic dermatitis. Such effects are obtained due to a fact that the external medicine of Embodiment 1 (and the external medicine of Embodiment 2) causes efficient percutaneous absorption of the active ingredient (sirolimus and its derivative). Therefore, a person skilled in the art who reads the specification can easily understand that these external medicines are applicable to "any skin disease caused by activation of mTOR". The skin disease to which the external medicine of Embodiment 1 is applicable includes not only diseases which occur in a skin tissue such as epidermis, dermis, and a subcutaneous tissue, but also diseases which occur in a blood vascular system in a skin tissue. Such a skin disease is not limited in particular. Examples of the skin disease include skin tumor, atopic dermatitis, rosacea, keloid, pigmented macule, hypopigmented macule, abnormal vascular malformation in skin, benign vascular tumor in skin, and epithelial nevus.

The skin tumor is preferably at least one tumor selected from the group consisting of skin tumor of tuberous sclerosis complex, seborrheic keratosis, and skin tumor of Recklinghausen's disease. The abnormal vascular malformation in skin is preferably hemangioma simplex, spider angioma, or angiokeratoma. The benign vascular tumor in skin is preferably strawberry hemangioma or senile angioma. The epithelial nevus is preferably at least one selected from the group consisting of verrucous nevus, systematized nevus, inflammatory linear verrucous epidermal nevus, and sebaceous nevus. The pigmented macule is preferably brownish pigmented macule, and the brownish pigmented macule is more preferably nevus spilus or café-au-lait spots. The hypopigmented macule is preferably vitiligo.

The external medicine of Embodiment 1 is thus capable of treating various types of skin diseases. Note that it is the inventors of the present invention that first found that atopic dermatitis, vitiligo, rosacea, keloid, and a skin tumor of seborrheic keratosis, is treatable by sirolimus and its derivative. This finding has not been reported so far.

As an external medicine for atopic dermatitis, a steroid and an ointment which contains tacrolimus are known. The steroid causes a side effect in a case of being used to treat atopic dermatitis. On the other hand, the ointment containing tacrolimus does not cause the side effect caused by the steroid, but causes a side effect of causing a tumor to be developed. The external medicine of Embodiment 1 (i) does not cause the side effect caused by the steroid, (ii) has an effect similar to that of the ointment containing tacrolimus, and (iii) further has an antitumor effect. Therefore, in a case where the external medicine of Embodiment 1 is used to treat atopic dermatitis, it is possible to avoid the side effect of the ointment containing tacrolimus. That is, the external medicine of Embodiment 1 is an external medicine for atopic dermatitis, which is safer than the ointment containing tacrolimus. Atopic dermatitis, which has been increasing in recent years, is a disease whose onset frequency is extremely high, and requires long term treatment. This causes a decrease in QOL of a patient.

Vitiligo, which is a subject of treatment using the external medicine of Embodiment 1, is not limited to congenital vitiligo such as vitiligo of tuberous sclerosis complex, and also includes acquired vitiligo such as vitiligo vulgaris. The number of patients with congenital vitiligo in Japan is approximately 50,000. One third of the patients are patients with vitiligo of tuberous sclerosis complex. There is no treatment for vitiligo of tuberous sclerosis complex at present. Therefore, it is revolutionary that the external medicine of Embodiment 1 is capable of treating vitiligo of tuberous sclerosis complex.

Further, the number of patients with acquired vitiligo is approximately 200,000. Of these, 75 % to 80 % are patients with vitiligo vulgaris. Vitiligo vulgaris is not a life-threatening disease. However, a patient with vitiligo vulgaris suffers a decrease in QOL by being affected by this disease. As treatment for vitiligo vulgaris, an ointment of a steroid or of vitamin D is externally applied. However, treatment effects of such ointments are subtle and, therefore, satisfying treatment results cannot be obtained. In view of this, irradiation with an excimer laser beam, PUVA (a treatment using methoxypsoralen and an ultraviolet ray A), nbUVB (narrow band UVB), or the like is carried out so as to treat vitiligo vulgaris. In this case, it is necessary to go to hospital so as to undergo the irradiation with such a laser beam or ultraviolet rays. Furthermore, there is a risk of carcinogenesis due to the irradiation with such a laser beam or ultraviolet rays. According to the external medicine of Embodiment 1, it is possible to effectively treat vitiligo vulgaris without such necessity and a risk.

The number of patients with rosacea is considerably high, and an onset frequency of rosacea is particularly high in a foreign country. Rosacea causes a decrease in QOL of a patient with rosacea in a case where a symptom due to rosacea becomes worse. Therefore, treatment for rosacea has been desired. However, there has been no effective therapeutic agent for rosacea. In view of this, the external medicine of Embodiment 1, which is capable of treating rosacea, has a significant influence in the pharmaceutical industry.

A benign tumor of skin (e.g., seborrheic keratosis), which is not a life-threatening disease, is a pathological change which appears in all humans according to age. The benign tumor causes a decrease in QOL of a patient with the benign tumor in a case where a symptom due to the benign tumor is serious.

As described above, atopic dermatitis, vitiligo, rosacea, and the benign tumor of skin causes a decrease in QOL of a patient, and further causes a decrease in labor productivity of the patient. It is possible to treat such diseases without causing a side effect by use of the external medicine of Embodiment 1. Therefore, it is possible to increase labor productivity of the patient. That is, the external medicine of Embodiment 1 is socioeconomically and medical-economically helpful.

As described above, the external medicine of Embodiment 1, the gel composition, and the kit for preparing the external medicine (the gel composition) of Embodiment 1 can each include another component other than the active ingredient, the solvent which dissolves the active ingredient, and the component which induces gelation. Further, the composition for preparing the external medicine (the gel composition) of Embodiment 1 can include another component other than the active ingredient and the solvent which dissolves the active ingredient. Examples of such components include water-soluble polymer, a pH adjuster, water, and other active ingredient effective in treating a target skin disease.

Examples of such water-soluble polymer include polyethylene glycol, starch, methyl cellulose, hydroxypropylcellulose (HPC), polyvinyl alcohol, polyvinyl methyl ether, and polyvinylpyrrolidone. Note that it is possible to increase adhesion of the external medicine of Embodiment 1 by mixing the external medicine of Embodiment 1 and hydroxypropylcellulose together. That is, by mixing the external medicine of Embodiment 1 and hydroxypropylcellulose together, it is possible to make it difficult that the external medicine comes off skin.

Examples of the pH adjuster include tris(hydroxymethyl)aminomethane.

Examples of the other active ingredient effective in treating a skin disease include a steroid and tacrolimus.

In a case where the external medicine of Embodiment 1, the gel composition, and the composition for preparing the external medicine (the gel composition) of Embodiment 1 include the foregoing another component, the total amount of the another component included in each of these can be determined as appropriate by a person skilled in the art with consideration for a point that the external medicine, to be produced, is in a gel form and a point that an effect of the active ingredient is not suppressed. For example, the total amount of the another component included in the external medicine is 49% by mass relative to the total mass of the external medicine. In a case where the kit for preparing the external medicine (the gel composition) includes the another component, the total amount of the another component can be any amount, provided that the total amount of the another component ultimately included in the external medicine produced is the above amount.

Further, the present invention provides a method for producing an external medicine for treating a skin disease. In a case where the external medicine of Embodiment 1 is one that is made up of the gel composition, the method can be a method for producing the gel composition. The method for producing the gel composition is simply required to include a step of producing the gel composition. The step of producing the gel composition is simply required to include gelation of the solution containing the active ingredient. Procedures for the gelation are, for example, such that (i) the active ingredient is dissolved in the solvent which dissolves the active ingredient and (ii) the resulting solution is mixed with the component which induces gelation. Note that, in a case where the another component is included in the gel composition, (i) the active ingredient and the another component are dissolved in the solvent which dissolves the active ingredient and (ii) the resulting solution is mixed with the component which induces gelation. Further, in a case where the external medicine of Embodiment 1 includes the gel composition and the another component, (i) the gel composition and the another component are mixed together by a conventionally known technique and (ii) a mixture thus obtained is caused to be in a gel dosage form.

The external medicine of Embodiment 1 is applicable to a human and a non-human animal. Examples of the "non-human animal" include mammals except a human, and birds. The mammals except a human are not limited in particular. Examples of the mammals except a human include: Artiodactyla such as cattle, a wild boar, a pig, a sheep, and a goat; Perissodactyla such as a horse; rodents such as a mouse, a rat, a hamster, and a squirrel; Lagomorpha such as a rabbit; and Carnivora such as a dog, a cat, and a ferret. Further, the birds are not limited in particular. Examples of the birds include a duck, a chicken, a pigeon, and a parakeet. Furthermore, the non-human animal is not limited to livestock or a companion animal (pet). The non-human animal can be a wild animal.

Further, the present invention provides a treatment method for the foregoing skin disease. The treatment method of the present invention is simply required to include a step of applying the external medicine of Embodiment 1 to an affected part of the skin disease. The wording "apply" in the specification indicates attaching the external medicine of Embodiment 1 to an affected part. The phrase "apply the external medicine to an affected part" includes, for example, administering the external medicine to an affected part, spraying the external medicine on an affected part, and attaching the external medicine on an affected part as a patch.

The external medicine (the gel composition) of Embodiment 1 can be arranged as below. Note, however, that the present invention is not limited to this.

For example, the external medicine (the gel composition) of Embodiment 1 can include Carbopol® 934NF, water, isopropanol, and tris(hydroxymethyl)aminomethane, in addition to the active ingredient.

In this case, a mass ratio of the active ingredient, Carbopol® 934NF, the water, isopropanol, and tris(hydroxymethyl)aminomethane can be 2-4 : 16 : 490-833 : 145-488 : 4. Note however that, according to the above mass ratio, a sum of (i) the mass ratio of the active ingredient, (i) the mass ratio of the water, and (iii) the mass ratio of isopropanol is 980.

More specifically, the mass ratio of the active ingredient, Carbopol® 934NF, the water, isopropanol, and tris(hydroxymethyl)aminomethane can be 2 : 16 : 833 : 145 : 4 (hereinafter, referred to as a ratio 1). Alternatively, the mass ratio of the active ingredient, Carbopol® 934NF, the water, isopropanol, and tris(hydroxymethyl)aminomethane can be 2 : 16 : 490 : 488 : 4 (hereinafter, referred to as a ratio 2). Comparison between the ratio 1 and the ratio 2 shows that the external medicine produced with the ratio 2 is more transparent than the external medicine produced with the ratio 1.

### [Embodiment 2: External medicine including an ointment composition]

An external medicine of Embodiment 2 includes an ointment composition containing an active ingredient for topically treating a skin disease. As used herein, the term "ointment composition" indicates an ointment composition in which the active ingredient is dispersed in a solvent which is not compatible with the active ingredient. It can be said that the external medicine including the ointment composition is an ointment.

As described above, according to the external medicine of the Embodiment 2, the active ingredient is dispersed in the solvent which is not compatible with the active ingredient. This increases absorption efficiency of the active ingredient into an affected part through skin. It is therefore possible to topically treat the skin disease by use of the active ingredient. Such dispersion of the active ingredient in the solvent may be performed, for example, by (i) dissolving the active ingredient in a solvent which dissolves the active ingredient and (ii) mixing a resulting solution with the solvent which is not compatible with the active ingredient.

The external medicine of Embodiment 2 can be one that is made up of the ointment composition. Alternatively, the external medicine of Embodiment 2 can further include the another component as described in Embodiment 1, provided that the external medicine maintains its ointment form. Similarly, the ointment composition can be one that is made up of the active ingredient, the solvent which dissolves the active ingredient, and the solvent which is not compatible with the active ingredient. Alternatively, the ointment composition can further include another component, provided that the ointment composition maintains its ointment form. As just described, according to the external medicine of Embodiment 2, the active ingredient is dispersed in the solvent which is not compatible with the active ingredient. The present invention also encompasses a composition which includes (i) the active ingredient, (ii) the solvent which dissolves the active ingredient, and (iii) the solvent which is not compatible with the active ingredient, the composition being in a state where the active ingredient has not yet dispersed in the solvent which is not compatible with the active ingredient. Similarly, the present invention also includes a kit in which the active ingredient, the solvent which dissolves the active ingredient, and the solvent which is not compatible with the active ingredient are separately included. Such composition and kit can be used to prepare the ointment composition (and the external medicine of Embodiment 2). As a matter of course, the composition for preparing the ointment composition can further include another component. The kit for preparing the ointment composition can further include another component.

Examples of the solvent which dissolves the active ingredient include, as described in Embodiment 1, isopropanol, ethanol, and propylene carbonate. In a case of Embodiment 2 (the ointment composition), propylene carbonate is preferable.

The solvent which is not compatible with the active ingredient is not limited in particular. However, examples of the solvent which is not compatible with the active ingredient include wax, paraffin, and petrolatum. Examples of wax include: natural wax such as white beeswax, lanolin, carnauba wax, and spermaceti wax; mineral wax such as montan wax; and synthetic wax. Examples of paraffin include liquid paraffin and solid paraffin. Examples of petrolatum include white petrolatum and yellow petrolatum.

According to Embodiment 2, the active ingredient is dissolved in the solvent which dissolves the active ingredient, and the resulting solution is mixed with the solvent which is not compatible with the active ingredient. This causes the active ingredient to be evenly dispersed as fine particles in the solvent which is not compatible with the active ingredient. Such dispersion remarkably increases absorption of the active ingredient into an affected part through skin. By using the external medicine of Embodiment 2, treatment of the skin disease is realized with a much smaller amount of sirolimus than an amount of sirolimus used in a conventional external medicine. Further, since the active ingredient is dispersed as described above, the active ingredient is likely to remain in an affected part without leaking into blood. With the use of the external medicine of Embodiment 2, it is possible to treat the skin disease without causing a side effect, unlike the conventional external medicine.

An amount of the active ingredient contained in the external medicine of Embodiment 2 is preferably 0.01% by mass to 2% by mass, more preferably 0.03% by mass to 1% by mass relative to the total mass of the external medicine. In a case where the amount of the active ingredient is within this range, it is possible to treat the skin disease without causing a side effect. Note that an amount of the active ingredient contained in the composition for preparing the external medicine is also similar to the amount of the active ingredient contained in the external medicine. An amount of the active ingredient included in the kit for preparing the external medicine can be any amount, provided that the amount of the active ingredient ultimately contained in the external medicine produced falls within the range. Further, an amount of the active ingredient contained in the ointment composition can be determined as appropriate by a person skilled in the art, provided that the amount of the active ingredient contained in the external medicine falls within the range.

An amount of the solvent which dissolves the active ingredient and which is contained in the external medicine (or the ointment composition) of Embodiment 2 is not limited in particular. It is only necessary that the amount of the solvent is enough to dissolve the active ingredient sufficiently. For example, the amount of the solvent which dissolves the active ingredient is 2 to 100 times larger than the amount of the active ingredient. The amount of the solvent is preferably 3 to 50 times larger, more preferably 5 to 29 times larger than the amount of the active ingredient. Note that an amount of the solvent which dissolves the active ingredient and which is contained in the composition for preparing the external medicine is similar to the amount of the solvent which dissolves the active ingredient and which is contained in the external medicine. An amount of the solvent which dissolves the active ingredient and which is included in the kit for preparing the external medicine can be any amount, provided that the amount of the solvent is enough to dissolve the active ingredient sufficiently.

An amount of the solvent which is not compatible with the active ingredient and which is contained in the external medicine (or the ointment composition) of Embodiment 2 is not limited in particular. It is only necessary that the amount of the solvent is enough for the active ingredient to be uniformly dispersed, as fine particles, in the solvent which is not compatible with the active ingredient. For example, the amount of the solvent which is not compatible with the active ingredient is 50 to 1000 times larger than the amount of the active ingredient. The amount of the solvent which is not compatible with the active ingredient is preferably 70 to 500 times larger, more preferably 94 to 470 times larger than the amount of the active ingredient. Note that an amount of the solvent contained in the composition for preparing the external medicine is similar to the amount of the solvent which is not compatible with the active ingredient and which is contained in the external medicine. An amount of the solvent which is not compatible with the active ingredient and which is included in the kit for preparing the external medicine can be any amount, provided that the amount of the solvent is enough for the active ingredient to be sufficiently dispersed.

As described earlier, the external medicine of Embodiment 2, the ointment composition, and the composition and the kit for preparing the external medicine (the ointment composition) of Embodiment 2 can each include another component other than the active ingredient, the solvent which dissolves the active ingredient, and the solvent which is not compatible with the active ingredient. As to the another component, the explanation in Embodiment 1 can be referred to. Therefore, the explanation on the another component will not be given here.

Further, the present invention provides a method for producing an external medicine for treating a skin disease. In a case where the external medicine of Embodiment 2 is one that is made up of the ointment composition, the method can be a method for producing the ointment composition. The method for producing the ointment composition is simply required to include a step of producing the ointment composition. The step of producing the ointment composition is simply required to include dispersing the active ingredient in the solvent which is not compatible with the active ingredient. Procedures for dispersing the active ingredient in the solvent which is not compatible with the active ingredient is such that (i) the active ingredient is dissolved in the solvent which dissolves the active ingredient and (ii) the resulting solution is mixed with the solvent which is not compatible with the active ingredient.

Specifically, the solution in which the active ingredient is dissolved is added to the solvent which is not compatible with the active ingredient, and then a mixture thus obtained is stirred with the use of Planetary Centrifugal Mixer manufactured by THINKY CORPORATION or HOMO MIXER manufactured by PRIMIX Corporation. This allows the active ingredient to be dispersed, in a form of emulsion, in the solvent which is not compatible with the active ingredient. By using such mixers, it is possible to uniform a particle size of the active ingredient and to successfully disperse the active ingredient, whose particle size is thus uniformed in, the solvent which is not compatible with the active ingredient. In a case where Planetary Centrifugal Mixer is used, the mixture may be stirred, for example, at room temperature at 2000 rpm for 1 minute, then at 1000 rpm for 5 minutes, and then at 500 rpm for 3 minutes. A condition of stir with the use of HOMO MIXER can be set as appropriate by a person skilled in the art based on an operating manual and the like accompanying the HOMO MIXER. Particularly, by using HOMO MIXER, it is possible to prepare the external medicine of Embodiment 2 in large numbers.

Note that, in a case where the solvent which is not compatible with the active ingredient is solid at room temperature, the solvent may be heated into a liquid state and then mixed with the solution in which the active ingredient is dissolved. For example, a component of any kind (wax, paraffin, or petrolatum), which is solid at room temperature, is melted by being heated to its melting point (for example, 70°C), and then mixed with the solution of the active ingredient. A resulting mixture is cooled down to near room temperature (for example, 40°C), and then added to the solution in which the active ingredient is dissolved. The mixture is stirred as described above, so that the ointment composition is produced.

In a case where the another component is contained in the ointment composition, (i) the active ingredient and the another component are dissolved in the solvent which dissolves the active ingredient and (ii) the resulting solution is mixed with the solvent which is not compatible with the active ingredient. Further, in a case where the external medicine of Embodiment 2 contains the ointment composition and the another component, (i) the ointment composition and the another component are mixed together by a conventionally known technique and (ii) a mixture thus obtained is formulated in an ointment dosage form.

Note that features of the present invention, other than the features described in Embodiment 2 (the active ingredient, the kit, the another component, the disease to which the external medicine is applied, the animal to which the external medicine is applied, the method for treating the skin disease, etc.), are identical to the features described in Embodiment 1, or can be ones that a person skilled in the art applies as appropriate by altering the features described in Embodiment 1.

The external medicine (the ointment composition) of Embodiment 2 can be arranged as below. Note, however, that the present invention is not limited to this.

For example, the external medicine (the ointment composition) of Embodiment 2 can contain propylene carbonate, solid paraffin, and white petrolatum, in addition to the active ingredient. The external medicine (the ointment composition) of Embodiment 2 can also contain liquid paraffin, in addition to propylene carbonate, solid paraffin, and white petrolatum. The external medicine (the ointment composition) of Embodiment 2 can also contain white beeswax, in addition to propylene carbonate, solid paraffin, white petrolatum, and liquid paraffin.

In this case, a mass ratio of the active ingredient, propylene carbonate, solid paraffin, white petrolatum, liquid paraffin, and white beeswax can be 0.3-10 : 50-59.4 : 30-45 : 895 : 0-10 : 0-5. Note however that, according to the above mass ratio, a sum of (i) the mass ratio of the active ingredient, (ii) the mass ratio of propylene carbonate, (iii) the mass ratio of solid paraffin, and (iv) the mass ratio of liquid paraffin is 105.

More specifically, the mass ratio of the active ingredient, propylene carbonate, solid paraffin, white petrolatum, liquid paraffin, and white beeswax can be also 2 : 58 : 30 : 895 : 10 : 5 (hereinafter, referred to as a ratio 3). Further, the mass ratio of the active ingredient, propylene carbonate, solid paraffin, white petrolatum, liquid paraffin and white beeswax can be 2 : 58 : 45 : 895 : 0 : 0 (hereinafter, referred to as a ratio 4). Furthermore, the mass ratio of the active ingredient, propylene carbonate, solid paraffin, white petrolatum, liquid paraffin and white beeswax can be 2 : 58 : 35 : 895 : 10 : 0 (hereinafter, referred to as a ratio 5). In a case where the ratio 3 and the ratio 4 are compared with each other, the external medicine produced with the ratio 3 is more transparent and includes less water in its surface than the external medicine produced with the ratio 4. In a case where the ratio 3 and the ratio 5 are compared with each other, the external medicine produced with the ratio 3 is smoother and includes less water in its surface than the external medicine produced with the ratio 5.

The present invention can be also arranged as below.

The gel composition is preferably a gelated solution containing sirolimus and/or a derivative thereof.

According to the present invention, the solution is preferably a dissolving solution in which sirolimus and/or a derivative thereof is dissolved in isopropanol and/or ethanol.

The skin disease to which the present invention is applied is preferably at least one disease selected from the group consisting of skin tumor, atopic dermatitis, rosacea, keloid, pigmented macule, hypopigmented macule, abnormal vascular malformation in skin, benign vascular tumor in skin, and epithelial nevus. The skin tumor is more preferably at least one tumor selected from the group consisting of skin tumor of tuberous sclerosis complex, seborrheic keratosis, and skin tumor of Recklinghausen's disease. The abnormal vascular malformation in skin is preferably hemangioma simplex, spider angioma, or angiokeratoma. The benign vascular tumor in skin is preferably strawberry hemangioma or senile angioma. The epithelial nevus is preferably at least one selected from the group consisting of verrucous nevus, systematized nevus, inflammatory linear verrucous epidermal nevus, and sebaceous nevus. The pigmented macule is preferably brownish pigmented macule. The brownish pigmented macule is more preferably nevus spilus or café-au-lait spots. The hypopigmented macule is preferably vitiligo.

The gel composition preferably contains at least one of Carbopol® 934NF, water, isopropanol, and tris(hydroxymethyl)aminomethane.

The gel composition is preferably such that a mass ratio of (i) sirolimus and/or a derivative thereof, (ii) Carbopol® 934NF, (iii) water, (iv) isopropanol, and (iv) tris(hydroxymethyl)aminomethane is 2-4 : 16 : 490-833 : 145-488 : 4; and a sum of (i) the mass ratio of sirolimus and/or the derivative thereof, (ii) the mass ratio of water, and (iii) the mass ratio of isopropanol is 980.

The gel composition is preferably such that a mass ratio of (i) sirolimus and/or a derivative thereof, (ii) Carbopol® 934NF, (iii) water, (iv) isopropanol, and (iv) tris(hydroxymethyl)aminomethane is 2 : 16 : 490 : 488 : 4.

The ointment composition preferably contains at least one of propylene carbonate, solid paraffin, white petrolatum, liquid paraffin, and white beeswax.

The ointment composition is preferably such that a mass ratio of (i) sirolimus and/or a derivative thereof, (ii) propylene carbonate, (iii) solid paraffin, (iv) white petrolatum, (v) liquid paraffin, and (vi) the white beeswax is 0.3-10 : 50-59.4 : 30-45 : 895 : 0-10 : 0-5; and a sum of (i) the mass ratio of sirolimus and/or the derivative thereof, (ii) the mass ratio of propylene carbonate, (iii) the mass ratio of the solid paraffin, (iv) the mass ratio of the liquid paraffin, and (v) the mass ratio of the white beeswax is 105.

The ointment composition is preferably such that a mass ratio of (i) sirolimus and/or a derivative thereof, (ii) propylene carbonate, (iii) solid paraffin, (iv) white petrolatum, (v) liquid paraffin, and (vi) the white beeswax is 2 : 58 : 30 : 895 : 10 : 5.

A composition for preparing an external medicine for topically treating a skin disease of the present invention includes sirolimus and/or a derivative thereof.

Further, a kit for preparing an external medicine for topically treating a skin disease of the present invention includes sirolimus and/or a derivative thereof.

Further, a method for producing an external medicine of the present invention includes the step of preparing a gel composition or an ointment composition, each of which contains sirolimus and/or a derivative thereof. The solution can be prepared with the use of the composition or the kit.

The present invention is not limited to the embodiments above, but may be altered by a skilled person within the scope of the claims. The following description will more specifically discuss the present invention with Example.

### Example

### [Preparation of external medicines of the present invention]

### <Preparation Example 1: Preparation of a gel containing 0.4% by mass of sirolimus>

4 mg of a reagent of sirolimus (produced by Calbiochem; 553210) was dissolved in 486 mg of isopropanol (produced by Wako Pure Chemical Industries, Ltd.; 166-04836). 490 mg of a melt thus obtained was mixed with a mixture of 16 mg of Carbopol® 934NF Polymer (produced by Lubrizol Corporation) and 490 mg of H₂O. A mixture obtained was mixed with 4 mg of tris(hydroxymethyl)aminomethane (produced by Wako Pure Chemical Industries, Ltd.; 203-06272). In this way, a gel (gel 1) containing 0.4% by mass of sirolimus was prepared.

### <Preparation Example 2: Preparation 1 of a gel containing 0.2% by mass of sirolimus>

A gel (gel 2) containing 0.2% by mass of sirolimus was prepared by carrying out the same operations as in Preparation Example 1, except that 2 mg of the reagent of sirolimus was dissolved in 488 mg of isopropanol.

### <Preparation Example 3: Preparation 2 of a gel containing 0.2% by mass of sirolimus>

A gel (gel 3) containing 0.2% by mass of sirolimus was prepared by carrying out the same operations as in Preparation example 2 except that Rapamune was used instead of the reagent of sirolimus. Specifically, 2 mg of a tablet of Rapamune was pulverized and sieved through a 75-µm mesh in order to remove impurities (a vehicle etc.), and a ground product thus obtained was dissolved in 488 mg of isopropanol.

### <Preparation Example 4: Preparation of an ointment containing 1% by mass of sirolimus>

10 mg of the reagent of sirolimus (produced by Calbiochem; 553210) was dissolved in 50 mg of propylene carbonate (produced by Wako Pure Chemical Industries, Ltd.; 168-04972). Then, 5 mg of white beeswax, 10 mg of liquid paraffin (produced by Wako Pure Chemical Industries, Ltd.; 128-04375), 30 mg of solid paraffin (produced by Wako Pure Chemical Industries, Ltd.; 415-25791), and 895 mg of white petrolatum (propet) (produced by Maruishi Pharmaceutical Co. Ltd, Merck & Co., Inc., or Astra Japan) were mixed together at 70°C to be dissolved.

A mixture thus obtained was cooled down to 40°C, and then the above-described melt of the reagent of sirolimus and propylene carbonate was added in the mixture. A resultant mixture was stirred with use of Planetary Centrifugal Mixer (manufactured by THINKY CORPORATION: Nano Pulverizer NP-100) at normal temperature at 2000 rpm for 1 minute, then at 1000 rpm for 5 minutes, and then at 500 rpm for 3 minutes. Thus prepared was an ointment (ointment 1) containing 1% by mass of sirolimus.

Although Preparation Example 4 used Planetary Centrifugal Mixer, it is also possible to use HOMO MIXER manufactured by PRIMIX Corporation. The use of HOMO MIXER enables preparation of large amounts of the ointment.

### <Preparation Example 5: Preparation 1 of an ointment containing 0.2% by mass of sirolimus>

An ointment (ointment 2) containing 0.2% by mass of sirolimus was prepared by carrying out the same operations as in Preparation Example 4 except that 2 mg of the reagent of sirolimus was dissolved in 58 mg of propylene carbonate.

### <Preparation Example 6: Preparation 2 of an ointment containing 0.2% by mass of sirolimus>

An ointment (ointment 3) containing 0.2% by mass of sirolimus was prepared by carrying out the same operations as in Preparation Example 5 except that Rapamune (produced by Pfizer Inc.; 29269) was used instead of the reagent of sirolimus. Specifically, 2 mg of a tablet of Rapamune was pulverized and sieved through a 75-µm mesh in order to remove impurities, and a ground product thus prepared was dissolved in 58 mg of propylene carbonate.

### <Reference Example 1: Preparation of an ointment containing 0.2% by mass of sirolimus with use of a commercially available ointment as a base>

An ointment (Reference Example 1) containing 0.2% by mass of sirolimus was prepared with use of, as a base, Protopic® ointment 0.03% for children's use (Astellas Pharma Inc.).

### <Reference Example 2: Preparation of another ointment containing 0.2% by mass of sirolimus with use of a commercially available ointment as a base>

An ointment (Reference Example 2) containing 0.2% by mass of sirolimus was prepared by carrying out the same operations as in Reference Example 1 except that Protopic® ointment 0.1% was used instead of Protopic® ointment 0.03% for children's use.

### [Checking amounts of external medicines of the present invention absorbed via artificial skin]

In the present example, the following test was conducted to check the amounts of external medicines of the present invention absorbed via artificial skin.

### <Test method>

The above-described external medicines of the present invention (the ointments 2 and 3 and the gels 2 and 3) were each applied to skin pieces in transwells of TESTSKIN® LSE-d and to skin pieces in transwells of TESTSKIN® LSE-high (TOYOBO). An absorption surface in a ring had a diameter of 1 cm.

Each transwell was moved and placed on an assay tray, to which a culture medium (TESTSKIN® LSE® ASSAY MEDIUM (TOYOBO)) had been added. The transwell was incubated in an incubator for 24 hours. The temperature and the CO₂ concentration inside the incubator were 37°C and 5%, respectively.

After the incubation, the external medicine on a surface of the transwell was wiped off with a sheet of filter paper, and the surface was washed with a culture medium. In this manner, the external medicine remaining on the surface was eliminated.

The transwell was placed on a heat tray covered with a plastic wrap. The heat tray was set to 60°C and heated the transwell for 60 seconds to 90 seconds.

A tissue was extracted from the transwell. From the tissue, an epidermis was removed and then a dermal layer was collected. The collected dermal layer was put in an Eppendorf tube.

The concentration of sirolimus in the dermal layer was measured with use of an LC/ESI/MS system. HPLC P4000, AS3000 thermostat (Thermo Fisher KK, San Jose, CA, USA) was used as the LC/ESI-MS system. LCQ Finigan Mat (Thermo Fisher KK, San Jose, CA, USA) was used as a detector of ESI/MS. HTC-Pal (AMR Inc, Tokyo, Japan) was used as an autosampler. For data analysis, X caliber ver1.2 (Thermo Fisher KK, San Jose, CA, USA) was used.

The above-described test method was carried out 3 times for each of the external medicines, and an average value and a standard deviation of concentrations of sirolimus in the dermal layers were calculated. Whether there was a statistically significant difference between results of the tests obtained with use of the respective external medicines was checked by calculating a p value with use of a Student's t-test having no correspondence. In a case where the p value was less than 0.05, a statistically significant difference was recognized.

### <Test results>

Fig. 1 shows test results obtained with use of the gel 2, the ointment 2, and Reference Example 1. As shown in Fig. 1, the concentration of sirolimus in dermis was: 8.08 ± 3.85 ng/mg in the case of using the gel 2; 10.03 ± 2.68 ng/mg in the case of using the ointment 2; and 1.82 ± 0.319 ng/mg in the case of using an ointment base of tacrolimus. A p value between the gel 2 and Reference Example 1 and a p value between the ointment 2 and Reference Example 1 were both less than 0.05. Based on the p values, it was confirmed that there was a significant difference between the gel 2 and Reference Example 1 and between the ointment 2 and Reference Example 1. It was thus found that the gel 2 and the ointment 2 could each cause an active ingredient to permeate skin more efficiently than Reference Example 1 did.

Fig. 2 shows test results obtained with use of the gel 2, the gel 3, the ointment 2, and the ointment 3. As shown in (a) through (d) of Fig. 2, the concentration of sirolimus in dermis was: 1.823 ± 0.146 ng/mg in the case of using the gel 2; 1.073 ± 0.199 ng/mg in the case of using the gel 3; 1.087 ± 0.073 ng/mg in the case of using the ointment 2; and 0.26 ± 0.098 ng/mg in the case of using the ointment 3.

As shown in (a) of Fig. 2, a p value between the gel 2 and the ointment 2 was less than 0.05, and it was thus confirmed that there was a significant difference between the gel 2 and the ointment 2. As shown in (b) of Fig. 2, a p value between the gel 3 and the ointment 3 was less than 0.05, and it was thus confirmed that there was a significant difference between the gel 3 and the ointment 3. Based on the results, it was found that the gels could cause sirolimus to permeate skin more efficiently than the ointments did. This appears to be because sirolimus was dissolved in a solvent (isopropanol) in the processes of preparing the gels. Accordingly, it appears to be preferable that (i) an ointment be used for a lesion (atopic dermatitis or the like) that has many eroded parts, absorbs an external medicine well, and is easily stimulated and (ii) a gel be used for a lesion (vitiligo or the like) that does not absorb an external medicine well or get stimulated easily.

As shown in (c) of Fig. 2, a p value between the gel 2 and the gel 3 was less than 0.05, and it was thus confirmed that there was a significant difference between the gel 2 and the gel 3. As shown in (d) of Fig. 2, a p value between the ointment 2 and the ointment 3 was less than 0.05, and it was thus confirmed that there was a significant difference between the ointment 2 and the ointment 3. Based on these results, it was found that an external medicine prepared with use of sirolimus in the form of a reagent could cause sirolimus to permeate skin more efficiently than an external medicine prepared with use of sirolimus in the form of a tablet (Rapamune) did. Accordingly, it was found that using sirolimus in the form of a reagent makes it possible to prepare a more effective external medicine.

### [Checking amount of sirolimus absorbed into skin of a mouse after an external medicine in the form of an ointment was applied to the skin of the mouse multiple times]

### <Test method>

In accordance with the above-described Preparation Example 5, an ointment (ointment 4) containing 0.03% by mass of sirolimus, an ointment (ointment 5) containing 0.06% by mass of sirolimus, and an ointment (ointment 6) containing 0.1% by mass of sirolimus were prepared.

The ointment 3 prepared with use of Rapamune, and the ointments 2, 4, 5, and 6, each of which had been prepared with use of the reagent of sirolimus, were each applied to skin of a mouse multiple times, and the amount of sirolimus absorbed into the skin was measured. Specifically, the ointments were each applied to skin of a mouse twice a week for 4 weeks. After 4 weeks, the skin of the mouse was collected and the amount of sirolimus in the skin was measured by an LC-ESI/MS method.

### <Test results>

The amount of sirolimus in dermis was: 294.2 ± 100.7 pg/mL in the case of using the ointment 3 (0.2% by mass); 333.1 ± 123 pg/mL in the case of using the ointment 2 (0.2% by mass); 67.59 ± 22.1 pg/mL in the case of using the ointment 4 (0.03% by mass); 80.31 ± 20.6 pg/mL in the case of using the ointment 5 (0.06% by mass); and 103.06 ± 22.3 pg/mL in the case of using the ointment 6 (0.1% by mass).

In the above-described test of applying the external medicines to the respective artificial skin pieces once, it was found that an external medicine prepared with use of sirolimus in the form of a reagent could cause sirolimus to permeate skin more efficiently than an external medicine prepared with use of sirolimus (Rapamune) in the form of a tablet did. On the other hand, it was found that applying the ointment 3, which had been prepared with use of sirolimus in the form of a tablet, to skin of a mouse multiple times allows the amount of the ointment 3 absorbed into dermis to be increased closer to that of the ointment 2 prepared with use of the reagent of sirolimus. Therefore, even with an ointment prepared with use of sirolimus in the form of a tablet, applying the ointment multiple times makes it possible to deliver, to an affected part, sirolimus in an amount sufficient to treat a disease. This is likely to apply to not only ointments but also gels.

### [Treatment of angiofibroma in a patient with tuberous sclerosis complex with use of external medicines of the present invention]

In the present example, it was confirmed, by carrying out the following test, that external medicines of the present invention had an effect on a skin tumor of tuberous sclerosis complex. The external medicines of the present invention are therefore expected to have an effect also on a benign skin tumor (seborrheic keratosis or the like) other than tuberous sclerosis complex.

### <Test method>

Patients with tuberous sclerosis complex were divided into a group of patients (four people) to be treated with use of the ointment 3 and a group of patients (eight people) to be treated with use of the gel 3. To each patient, the external medicine was applied twice a day for 12 weeks. Specifically, the external medicine was applied to angiofibroma on one of the right and left sides of the face of the patient, and only a base (control) containing no active ingredient (sirolimus) was applied to angiofibroma on the other of the right and left sides of the face. The treatment was stopped 12 weeks after the start of the treatment. During 3 months after the treatment was stopped, the progress of angiofibroma was followed. One of the patients who were treated with the gel 3 failed to appropriately make external use of the gel 3 as instructed, so that the patient was excluded during the test.

Before the start of the treatment and 12 weeks after the start of the treatment, angiofibroma was photographed and evaluated. The evaluation of angiofibroma was carried out by assigning a score on a 4-point scale with respect to (i) degree of alleviation of erubescence, (ii) degree of reduction in size of papules, (iii) degree of flatness of a surface of an affected part, and (iv) overall viewpoint (an overall score).

That is, with respect to the alleviation of erubescence, the score 0 was given in a case where erubescence had not been alleviated as compared to before the start of the treatment, the score 1 was given in a case where erubescence had been alleviated by 49% as compared to before the start of the treatment, the score 2 was given in a case where erubescence had been alleviated by 50% to 80% as compared to before the start of the treatment, and the score 3 was given in a case where erubescence had been alleviated by more than 80% as compared to before the start of the treatment.

With respect to the reduction in size of papules, the score 0 was given in a case where papules was not reduced in size as compared to before the start of the treatment, the score 1 was given in a case where papules was reduced in size by 49% as compared to before the start of the treatment, the score 2 was given in a case where papules was reduced in size by 50% to 80% as compared to before the start of the treatment, and the score 3 was given in a case where papules was reduced in size by more than 80% as compared to before the start of the treatment.

With respect to the flatness of a surface of an affected part, the score 0 was given in a case where an affected part was not flatter as compared to before the start of the treatment, the score 1 was given in a case where an affected part was flatter by 49% as compared to before the start of the treatment, the score 2 was given in a case where an affected part was flatter by 50% to 80% as compared to before the start of the treatment, and the score 3 was given in a case where an affected part was flatter by more than 80% as compared to before the start of the treatment.

With respect to the overall score, an average of a sum of a score for erubescence, a score for papules, and a score for a surface of an affected part was used as an overall score.

### <Test results>

Figs. 3 through 5 each show (i) a photograph of angiofibroma taken before the start of the treatment with the gel 3 and (ii) a photograph of angiofibroma taken 12 weeks after the start of the treatment. As shown in Figs. 3 through 5, the gel 3 was applied to angiofibroma on the left cheek of a patient, and the control was applied to angiofibroma on the right cheek of the patient.

(a) of Fig. 3, (a) of Fig. 4, and (a) of Fig. 5 are each a photograph of angiofibroma taken before the start of the treatment with the gel 3, and (b) of Fig. 3, (b) of Fig. 4, and (b) of Fig. 5 are each a photograph of angiofibroma taken 12 weeks after the start of the treatment with the gel 3. (c) and (d) of Fig. 3 are enlarged views of the left cheeks shown in (a) and (b) of Fig. 3, respectively. As is clear from a comparison between (a) and (b) of Fig. 3, a comparison between (c) and (d) of Fig. 3, a comparison between (a) and (b) of Fig. 4, and a comparison between (a) and (b) of Fig. 5, angiofibroma to which the gel 3 had been applied was alleviated, whereas angiofibroma to which the control had been applied exhibited no change.

Fig. 6 through 7 each show (i) a photograph of angiofibroma taken before the start of the treatment with the ointment 3 and (ii) a photograph of angiofibroma taken 12 weeks after the start of the treatment. As shown in Fig. 6, the ointment 3 was applied to angiofibroma on the left cheek of a patient, and the control was applied to angiofibroma on the right cheek of the patient. As shown in Fig. 7, the ointment 3 was applied to angiofibroma on the right cheek of a patient, and the control was applied to angiofibroma on the left cheek of the patient.

(a) of Fig. 6 and (a) of Fig. 7 are each a photograph of angiofibroma taken before the start of the treatment with the ointment 3, and (b) of Fig. 6 and (b) of Fig. 7 are each a photograph of angiofibroma taken 12 weeks after the start of the treatment with the ointment 3. (c) and (d) of Fig. 6 are enlarged views of the left cheeks of (a) and (b) shown in Fig. 6, respectively. As is clear from a comparison between (a) and (b) of Fig. 6, a comparison between (c) and (d) of Fig. 6, and a comparison between (a) and (b) of Fig. 7, angiofibroma to which the ointment 3 had been applied was alleviated, whereas angiofibroma to which the control had been applied exhibited no change.

Evaluation results as described above of angiofibroma are shown in Figs. 8 through 9.

Fig. 8 is a graph showing scores given to the respective patients 12 weeks after the start of the treatment with the gel 3 or the control. (a) of Fig. 8 is a graph showing overall scores. (b) of Fig. 8 is a graph showing scores with respect to erubescence. (c) of Fig. 8 is a graph showing scores with respect to papules. (d) of Fig. 8 is a graph showing scores with respect to a surface of an affected part. (a) through (d) of Fig. 8 each show, on the left-hand side thereof, scores obtained by the application of the gel 3 and, on the right-hand side thereof, scores obtained by the application of the control.

As shown in (a) of Fig. 8, (i) in the case of using the gel 3, a patient 1, a patient 2, a patient 3, and a patient 4 had a score of 4, 4, 4, and 3, respectively, and an average of the scores of the respective patients 1 through 4 was 3.75, and (ii) in the case of using the control, the patients 1 through 4 each had a score of zero. As shown in (b) of Fig. 8, (i) in the case of using the gel 3, the patient 1, the patient 2, the patient 3, and the patient 4 had a score of 4, 4, 4, and 3, respectively, and an average of the scores of the respective patients 1 through 4 was 3.75, and (ii) in the case of using the control, the patients 1 through 4 each had a score of zero. As shown in (c) of Fig. 8, (i) in the case of using the gel 3, the patient 1, the patient 2, the patient 3, and the patient 4 had a score of 3, 3, 4, and 2, respectively, and an average of the scores of the respective patients 1 through 4 was 3, and (ii) in the case of using the control, the patients 1 through 4 each had a score of zero. As shown in (d) of Fig. 8, (i) in the case of using the gel 3, the patient 1, the patient 2, the patient 3, and the patient 4 each had a score of 4, and an average of the scores of the respective patients 1 through 4 was 4, and (ii) in the case of using the control, the patients 1 through 4 each had a score of zero.

In each of (a) through (d) of Fig. 8, whether there was a statistically significant difference between the average of the scores obtained in the case of using the gel 3 and the average of the scores obtained in the case of using the control was confirmed by calculating a p value with use of a Student's t-test having a correspondence. The p values thus obtained were each less than 0.05, and it was thus found that there were statistically significant differences.

Fig. 9 is a graph showing scores given to the respective patients 12 weeks after the start of the treatment with the ointment 3 or the control. (a) of Fig. 9 is a graph showing overall scores. (b) of Fig. 9 is a graph showing scores with respect to erubescence. (c) of Fig. 9 is a graph showing scores with respect to papules. (d) of Fig. 9 is a graph showing scores with respect to a surface of an affected part. (a) through (d) of Fig. 9 each show, on the left-hand side thereof, scores obtained by the application of the ointment 3 and, on the right-hand side thereof, scores obtained by the application of the control.

As shown in (a) of Fig. 9, (i) in the case of using the ointment 3, a patient 1, a patient 2, a patient 3, and a patient 4 had a score of 4, 4, 2, and 1.5, respectively, and an average of the scores of the respective patients 1 through 4 was 2.9, and (ii) in the case of using the control, the patients 1 through 4 each had a score of zero. As shown in (b) of Fig. 9, (i) in the case of using the ointment 3, the patient 1, the patient 2, the patient 3, and the patient 4 had a score of 4, 4, 2, and 1.5, respectively, and an average of the scores of the respective patients 1 through 4 was 2.9, and (ii) in the case of using the control, the patients 1 through 4 each had a score of zero. As shown in (c) of Fig. 9, (i) in the case of using the ointment 3, the patient 1, the patient 2, the patient 3, and the patient 4 had a score of 3, 1.5, 1, and 1, respectively, and an average of the scores of the respective patients 1 through 4 was 1.7, and (ii) in the case of using the control, the patients 1 through 4 each had a score of zero. As shown in (d) of Fig. 9, (i) in the case of using the ointment 3, the patient 1, the patient 2, the patient 3, and the patient 4 each had a score of 4, 4, 2, and 1.5, and an average of the scores of the respective patients 1 through 4 was 2.9, and (ii) in the case of using the control, the patients 1 through 4 each had a score of zero.

In each of (a) through (d) of Fig. 9, whether there was a statistically significant difference between the average of the scores obtained in the case of using the ointment 3 and the average of the scores obtained in the case of using the control was confirmed by calculating a p value with use of a Student's t-test having a correspondence. The p values thus obtained were each less than 0.05, and it was thus found that there were statistically significant differences.

Based on the results, it was found that use of the gel 3 and the ointment 3 enables alleviation of angiofibroma.

Before the start of the treatment with the gel 3 or the ointment 3 and after the day of ending the treatment (that is, 12 weeks after the start of the treatment), blood of each of the patients was collected and tested to measure components in the blood. Results of blood tests carried out with respect to respective patients 1 and 2 are shown in the table below.

**[Table 1]**

| | Patient 1 treated with gel 3 | | Patient 2 treated with ointment 3 | |
|---|---|---|---|---|
| | Before start of treatment | After day of ending treatment | Before start of treatment | After day of ending treatment |
| WBC(×10³/µL) | 5.82 | 5.82 | 5.81 | 4.62 |
| RBC(×10⁶/µL) | 4.36L | 4.42 | 5.08 | 4.95 |
| Hb(g/dL) | 13.6L | 13.8 | 15.1 | 14.8 |
| Ht(%) | 40.1L | 40.6L | 44.1 | 43.0 |
| Plat(×10³/µL) | 212 | 180 | 323 | 299 |
| Neu(%) | 46.5 | 46.3 | 62.0 | 46.1 |
| Lym(%) | 43.6 | 38.8 | 30.5 | 41.3 |
| Eo(%) | 3.3 | 4.5 | 3.0 | 5.3 |
| ba(%) | 0.5 | 0.5 | 0.6 | 0.6 |
| Cr(mg/dL) | 0.63 | 0.61 | 0.39 | 0.48 |
| AST(U/L) | 25 | 21 | 17 | 17 |
| ALT(U/L) | 35 | 33 | 12 | 12 |
| γGTP(U/L) | 196↑ | 186↑ | 14 | 12 |
| Tchl(mg/dL) | 125↓ | 131↓ | 220 | 182 |
| TG(mg/dL) | 108 | 84 | 94 | 90 |
| Glu(mg/dL) | 93 | 102 | 110 | 106 |
| GRP(mg/dL) | > 0.04 | 0.05 | > 0.04 | > 0.04 |

"WBC" indicates the count of white blood cells. "RBC" indicates the count of red blood cells. "Hb" indicates a hemoglobin content. "Ht" indicates a hematocrit. "Plat" indicates platelets. "Neu" neutrophils. "Lym" indicates lymphocytes. "Eo" indicates eosinophils. "ba" indicates basophils. "Cr" indicates creatinine. "AST" indicates aspartate aminotransferase. "ALT" indicates alanine aminotransferase. "γGTP" indicates gamma-glutamyl transferase. "Tchl" indicates total chloresterol. "TG" indicates triglyceride. "Glu" indicates blood sugar. "CRP" indicates a C-reactive protein.

As shown in Table 1, the blood components of each of the patients did not change significantly between before the start of the treatment and 12 weeks after the start of the treatment.

Whether or not the skins to each of which the gel 3 or the ointment 3 had been applied had contact dermatitis was investigated 12 weeks after the start of the treatment. The investigation showed that the skin had no contact dermatitis.

Blood was collected from each of the patients 12 weeks after the start of the treatment so as to detect sirolimus in the blood by the LC-ESI-MS method. Sirolimus in the blood, however, could not be detected. Based on this, it was found that, in a case where the gel 3 or the ointment 3 was applied to skin, sirolimus could permeate dermis but was not released into blood.

The above-described facts that (i) there was no change in the blood components, (ii) contact dermatitis did not develop, and (iii) sirolimus was not released into blood, show that there was produced no side effect caused by the application of the gel and the ointment 33.

Based on the result, it was found that use of the external medicines of the present invention makes it possible to treat angiofibroma of tuberous sclerosis complex effectively without producing any side effects. This suggests that the external medicines of the present invention are also effective against other skin tumors (e.g., a benign tumor of skin) caused by mTOR dysregualtion. Further, the fact that angiofibroma of tuberous sclerosis complex on a face had been treated showed that the external medicines of the present invention had permeated dermis.

### [Treatment of vitiligo with use of external medicines of the present invention]

Development of vitiligo is caused by, for example, melanocytes ceasing to produce melanin. Proteins involved in melanin production in melanocytes are MITF, TYR, DCT, and TYRP1.

Vitiligo is known to be developed often in a patient having a disease (such as tuberous sclerosis complex) caused by activation of mTOR. The inventors consider that a mechanism of development of vitiligo in such a patient is as follows. Activation of mTOR in melanocytes of the patient increases activity of S6K1. The increase in the activity of S6K1 inhibits activity of PI3K. Since PI3K activates an MAPK signaling pathway as well as an Akt signaling pathway, the inhibition of the activity of PI3K inhibits the Akt signaling pathway and the MAPK signaling pathway. This inhibits activity of p38, which is downstream of the Akt signaling pathway, and activity of MEK, which is downstream of the MAPK signaling pathway. Consequently, expression and activation of MITF via p38 and MEK is inhibited.

In a case where expression of MITF is stopped and/or an activity of MITF is reduced, activities of TYR and TYRP1 are reduced. This stops melanin production in melanocytes. In this manner, vitiligo is developed.

The inventors considered that, since the activation of mTOR was involved in development of vitiligo, external medicines of the present invention were effective for treating vitiligo. The following test was therefore carried out in the present example to examine what effect sirolimus has on expression of the above-mentioned proteins. It was confirmed that activation of mTOR was involved in development of vitiligo. It was also confirmed that the external medicines of the present invention had an effect on vitiligo.

### <Confirmation of increases caused by sirolimus in expression levels of MITF, TYR, and TYRP1>

### (Test method)

1.4×10⁵ melanocytes were inoculated in wells of 6-well culture plate, and a medium 254 (produced by KURABO INDUSTRIES LTD.; M254500) was added to the wells. Subsequently, the plate was incubated for 24 hours at a temperature of 37°C in a 5% CO₂ environment.

Sirolimus was added to the respective wells so as to have a final concentration of 0 nM (that is, no sirolimus is added to a culture medium), 1 nmol/L, 10 nmol/L, and 20 nmol/L, respectively. The plates were incubated for 24 hours or 48 hours at a temperature of 37°C in a 5% CO₂ environment.

From the wells thus incubated, the medium 254 was removed. Then, an operation of adding PBS to the wells and removing the PBS from the wells was carried out three times to wash the melanocytes with the PBS.

To the wells from which the PBS had been removed, 175 µL of an RNA lysis buffer was added. The RNA lysis buffer is attached to a kit of an SV Total RNA Solution System (manufactured by Promega Corporation; Z3105).

Melanocytes were peeled off the plate with use of a cell scraper.

The RNA lysis buffer containing melanocytes was collected into a 1.5 mL Eppendorf tube. Then, RNAs were extracted from the collected buffer.

Real-time RT-PCR was carried out with use of the RNAs to detect expression of mRNAs of MITF, mRNAs of TYR, mRNAs of TYRP1, and mRNAs of GAPDH. An expression level of the mRNAs of MITF, an expression level of the mRNAs of TYR, and an expression level of the mRNAs of TYRP1 were each calculated as a relative expression level with respect to an expression level of the mRNA of GAPD.

This experiment was carried out three times to calculate an average value and a standard deviation of relative expression levels of mRNAs of each of MITF, TYR, and TYRP1. Whether or not there was a statistically significant difference between test results obtained with use of the respective external medicines was checked by calculating a p value with use of a Student's t-test having no correspondence. In a case where the p value was less than 0.05, a statistically significant difference was recognized.

To detect expression of the mRNAs of MITF, Tagman® Gene Expression Assays (produced by Applied biosystems, Inc.; Hs00165156_m1) was used. To detect expression of the mRNAs of TYR, TagMan® Gene Expression Assays (produced by Applied biosystems, Inc.; Hs00165976_m1). To detect expression of the mRNAs of TYRP1, TagMan® Gene Expression Assays (produced by Applied biosystems, Inc.; Hs00167051_m1) was used. To detect expression of the mRNAs of GAPDH, TagMan® Gene Expression Assays (produced by Applied biosystems, Inc.; Hs99999905_m1) was used.

### (Test results)

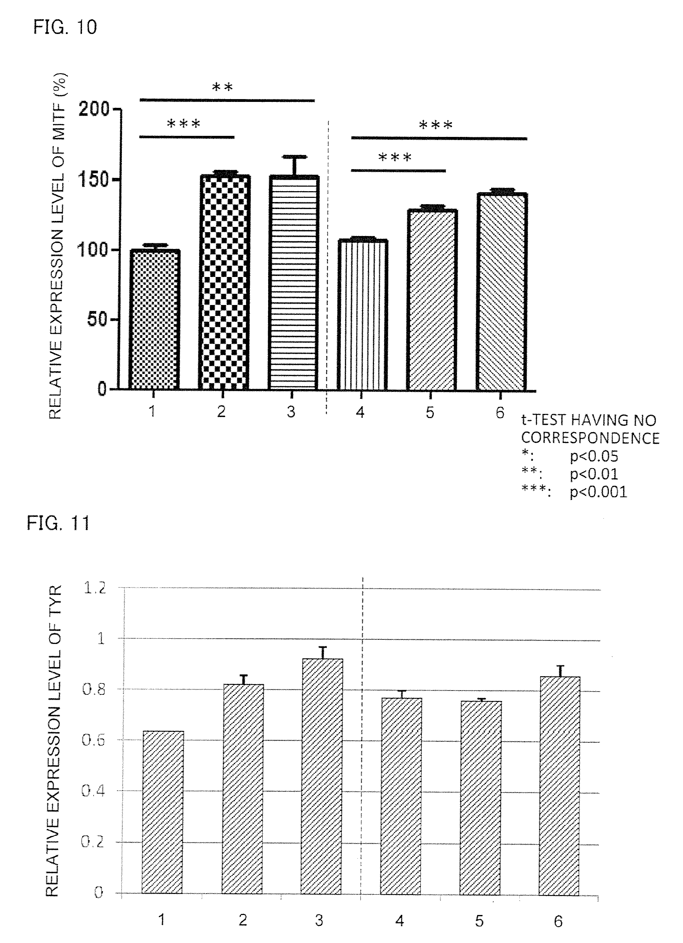

The test results are shown in Figs. 10 through 12.

Fig. 10 is a graph showing an average value and a standard deviation of relative expression levels of mRNAs of MITF. In Fig. 10, "1" indicates melanocytes which were incubated for 24 hours without being treated with sirolimus, "2" means melanocytes which were treated with 1 nM of sirolimus for 24 hours, "3" means melanocytes which were treated with 20 nM of sirolimus for 24 hours, "4" means melanocytes which were incubated for 48 hours without being treated with sirolimus, "5" means melanocytes which were treated with 1 nM of sirolimus for 48 hours, and "6" means melanocytes which were treated with 20 nM of sirolimus for 48 hours.

As shown in Fig. 10, "1" had relative expression levels of MITF of 100.00 ± 3.37, "2" had relative expression levels of MITF of 152.48 ± 3.47, "3" had relative expression levels of MITF of 152.18 ± 14.75, "4" had relative expression levels of MITF of 107.95 ± 1.38, "5" had relative expression levels of MITF of 129.41 ± 3.43, and "6" had relative expression levels of MITF of 141.56 ± 3.52. In Fig. 10, the sign "**" indicates that a p value between expression levels is less than 0.01, and the sign "***" indicates that a p value between expression levels is less than 0.001.

As shown in Fig. 10, the relative expression levels of the mRNAs of MITF in "2" and the relative expression levels of the mRNAs of MITF in "3" are each significantly higher than the relative expression levels of the mRNAs of MITF in "1". Similarly, the relative expression levels of the mRNAs of MITF in "5" and the relative expression levels of the mRNAs of MITF in "6" are each significantly higher than the relative expression levels of the mRNAs of MITF in "4". It was thus confirmed that treating melanocytes with 1 nM or 20 nM of sirolimus for 24 hours or 48 hours had increased expression levels of mRNAs.

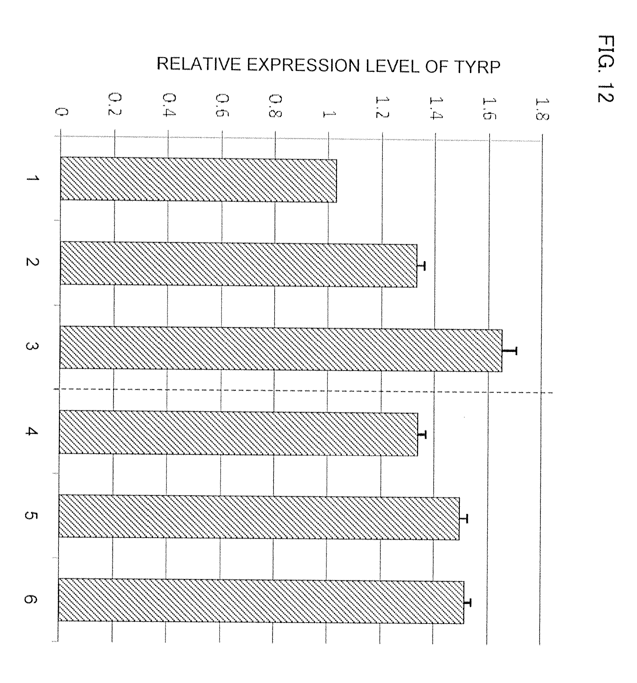

Figs. 11 and 12 are graphs respectively showing (i) an average value and a standard deviation of relative expression levels of mRNAs of TYR and (ii) an average and a standard deviation of relative expression levels of mRNAs of TYRP. In each of Figs. 11 and 12, "1" indicates melanocytes which were incubated for 24 hours without being treated with sirolimus, "2" means melanocytes which were treated with 1 nM of sirolimus for 24 hours, "3" means melanocytes which were treated with 20 nM of sirolimus for 24 hours, "4" means melanocytes which were incubated for 48 hours without being treated with sirolimus, "5" means melanocytes which were treated with 1 nM of sirolimus for 48 hours, and "6" means other melanocytes which were treated with 20 nM of sirolimus for 48 hours.

As shown in Fig. 11, "1" had relative expression levels of TYR of 0.640 ± 0.036, "2" had relative expression levels of TYR of 0.820 ± 0.047, "3" had relative expression levels of TYR of 0.921 ± 0.029, "4" had relative expression levels of TYR of 0.771 ± 0.009, "5" had relative expression levels of TYR of 0.760 ± 0.044, and "6" had relative expression levels of TYR of 0.858 ± 0.022.

As shown in Fig. 12, "1" had relative expression levels of TYRP of 1.031 ± 0.027, "2" had relative expression levels of TYRP of 1.333 ± 0.055, "3" had relative expression levels of TYRP of 1.652 ± 0.031, "4" had relative expression levels of TYRP of 1.338 ± 0.029, "5" had relative expression levels of TYRP of 1.450 ± 0.028, and "6" had relative expression levels of TYRP of 1.514 ± 0.124.

As shown in Fig. 11, it was confirmed that (i) treating melanocytes with 1 nM or 20 nM of sirolimus for 24 hours or (ii) treating melanocytes with 20 nM of sirolimus for 48 hours had increased expression levels of TYR. It was also confirmed that, as shown in Fig. 12, treating melanocytes with 1 nM or 20 nM of sirolimus for 24 hours or 48 hours had increased expression levels of TYRP.

The fact that treating melanocytes with sirolimus thus increases expression levels of respective MITF, TYR and TYRP indicates that production of melanin is increased.

### <Confirmation of an increase caused by sirolimus in an amount of production of melanin>

### (Test method)

Melanocytes (melanocytes derived from two normal people (hereinafter referred to as melanocytes 1 and melanocytes 2, respectively)) were inoculated in 6 wells. The number of melanocytes per well was 2×10⁵, and a medium 254/HMGS2 (PMA(-) (without antibiotics)) was used as a culture medium.

After 24 hours, sirolimus was added to each of the wells so as to have a concentration of 0 nM, 1 nM, or 10 nM, and was cultured for 4 days.

After the 4 days of culture, each of the wells was washed twice with PBS.

After being washed with PBS, the wells were each added 1 mL of an EDTA/ trypsin (2:1) solution so that melanocytes were peeled off the wells. The solution containing melanocytes was added into a 1.5-mL tube, and then the tube was subjected to centrifugation at 1500 rpm and 4°C for 5 minutes.

After the centrifugation, a supernatant in the tube was discarded, and then a pellet (melanocytes) was suspended in 1 mL of PBS. With use of 30 µL of a suspension thus obtained, melanocytes in the suspension was counted.

The other 970 µL of the suspension was subjected to centrifugation at 1500 rpm and 4°C for 5 minutes.

After the centrifugation, a supernatant in the tube was discarded to obtain a pellet, and 1 mL of NaOH (1N) was added to the pellet. A solution thus obtained was boiled at 100°C for 30 minutes and then cooled down to room temperature.

After being cooled down, the solution was subjected to centrifugation at 16,000 g for 20 minutes, and then an absorbance of a supernatant at 400 nm was measured. At the same time, absorbances of standards were also measured.

Various concentrations (1 µg/mL to 100 µg/mL) of melanin-containing NaOH were prepared by dissolving commercially available melanin in 1N NaOH. An absorbance of each of the various concentrations of melanin-containing NaOH was measured at 400 nm to prepare a standard curve.

From the standard curve, an amount of melanin contained in NaOH in which melanocytes had been dissolved was measured. The amount of melanin was corrected into an amount per 1×10⁴ cells.

### (Test results)

The corrected amount of melanin per 1×10⁴ cells is shown in Figs. 23 and 24. Fig. 23 shows data of the melanocytes 1, and Fig. 24 shows data of the melanocytes 2.

As is clear from Figs. 23 and 24, melanocytes treated with sirolimus had an increase in amount of production of melanin. Further, an amount of production of melanin increased as a concentration of sirolimus increased.

### <Confirmation of effects of external medicines of the present invention on vitiligo - 1 >

To vitiligo of a patient with tuberous sclerosis complex, the gel 3 or the ointment 3 was applied twice a day for 6 weeks. Fig. 13 shows a photograph (a) of vitiligo taken before the start of the application of the gel 3 and a photograph (b) of vitiligo taken 6 weeks after the start of the application of the gel 3. Fig. 14 shows a photograph (a) of vitiligo taken before the start of the application of the ointment 3 and a photograph (b) of vitiligo taken 6 weeks after the start of the application of the ointment 3. Arrows in Figs. 13 and 14 each show vitiligo. The vitiligo shown in (a) of Fig. 13 and the vitiligo shown in (a) of Fig. 14 have disappeared in (b) of Fig. 13 and (b) of Fig. 14, respectively. In addition, papules and red surfaces of the affected parts have also been alleviated. It was thus shown that vitiligo could be treated by application of the gel 3 or the ointment 3.

To vitiligo developed in a forehead region (a part which was exposed to light) or in an abdominal region (a part which was not exposed to light), the gel 3 or the ointment 3 was applied twice a day for 3 months. The part in which vitiligo was developed was examined with use of a spectrophotometer (a spectrophotometer manufactured by KONICA MINOLTA, INC.: M-2600d) to determine a symptom of vitiligo and check how the symptom of vitiligo underwent a change.

Fig. 25 shows data of four patients each having vitiligo developed in a forehead region (a part which is exposed to light). Fig. 26 shows data of four patients each having vitiligo developed in an abdominal region (a part which is not exposed to light).

As is clear from Figs. 25 and 26, vitiligo of each of the patients decreased in size as the time passed. That is, it was found that the external medicines of the present invention had therapeutic effects on vitiligo developed in various parts of a body. It was also found that the external medicines of the present invention each had a greater therapeutic effect on vitiligo developed in a forehead region (a part which was exposed to light) than on vitiligo developed in an abdominal region (a part which was not exposed to light).

### <Confirmation of effects of external medicines of the present invention on vitiligo - 2>

To vitiligo vulgaris (vitiligo vulgaris developed in a cervical region or an abdominal region), the gel 3 or the ointment 3 was applied twice a day for 2 months. Figs. 27 and 28 show photographs of vitiligo vulgaris of two patients. As is clear from Figs. 27 and 28, vitiligo vulgaris of each of the two patients has disappeared.

### [Treatment of atopic dermatitis with use of external medicines of the present invention]

Tacrolimus or pimecrolimus is used as a treatment drug for atopic dermatitis. Tacrolimus or the like is known to exhibit an effect on atopic dermatitis by binding to FKBP12 (immunophilin) so as to inhibit calcineurin. However, tacrolimus or the like has a side effect of causing a tumor to be developed.

On the other hand, sirolimus binds to FKBP12 similarly as tacrolimus but inhibits mTOR instead of calcineurin. Molecules inhibited by sirolimus and molecules inhibited tacrolimus are thus different from each other. It is known that inflammation via a mast cell is suppressed due to the inhibition of mTOR by sirolimus. Further, due to the inhibition of mTOR, an antitumor effect such as cell growth inhibition or apoptosis induction is exhibited. Based on the knowledge, the inventors considered that external medicines of the present invention were effective for treating atopic dermatitis. Unlike tacrolimus or the like, the external medicines of the present invention do not cause a tumor to be developed and can therefore be a safer therapeutic agent for atopic dermatitis. In view of this, in the present example, the following test was conducted to check an effect of the external medicines of the present invention on atopic dermatitis.

### <Test method>

An ointment (Biostir AD produced by Biostir Inc.; AD001) containing an antigen of mite was applied to skin of the backs of mice for 2 weeks to induce atopic dermatitis in the skin. Specifically, the mice were shaved on their backs so as to expose the skin of the backs. Then, the ointment containing the antigen of mite was applied to the exposed skin of the backs on the day of starting the application of the ointment (day 0), and on day 4, day 7, day 11, day 14, day 18, day 21, and day 25 after the day of starting the application.

On day 13 after the day of starting the application of the ointment containing the antigen of mite, (i) the ointment 2, (ii) an ointment (a control) identical to the ointment 2 except for containing no sirolimus, and (iii) an ointment (Protopic^{®} ointment 0.1%, a positive control) containing 0.1% by mass of tacrolimus were each applied, twice a week for 2 weeks, to a portion of skin in which atopic dermatitis had been induced. Specifically, on day 13, day 17, day 20, day 24, and day 27, the ointments were each applied to the portion of the skin. On day 0, day 7, day 13, day 14, day 17, day 21, day 24, and day 28, the portion of the skin to which each of the ointments was applied was observed so as to evaluate atopic dermatitis.

The evaluation of atopic dermatitis was carried out by assigning a 4-point score with respect to (i) degree of erythema, (ii) degree of edema, and (ii) degree of erosion.

That is, with respect to erythema, the score 0 was given in a case where erythema had not been alleviated as compared to before the start of the treatment, the score 1 was given in a case of mild erythema, the score 2 was given in a case of moderate erythema, and the score 3 was given in a case of severe erythema. With respect to edema, the score 0 was given in a case where edema had not been alleviated as compared to before the start of the treatment, the score 1 was given in a case of mild edema, the score 2 was given in a case of moderate edema, and the score 3 was given in a case of severe edema. With respect to erosion, the score 0 was given in a case where erosion had not been alleviated as compared to before the start of the treatment, the score 1 was given in a case of mild erosion, the score 2 was given in a case of moderate erosion, and the score 3 was given in a case of severe erosion. A sum of a score for erythema, a score for edema, and a score for erosion was used as an overall score.

On day 28, the mice were killed and a photograph of the back of each of the mice was taken. Skin in which atopic dermatitis had been induced was collected. Slices of the collected skin were prepared and were subjected to hematoxylin-eosin staining or toluidine blue staining.

### <Test results>

Results of the evaluation of atopic dermatitis are shown in Figs. 15 and 16. Fig. 15 shows graphs each showing overall scores given to respective three mice at each point in time of treatment with use of one of the above-described ointments. (a) of Fig. 15 is a graph showing overall scores of respective mice in the case of using the positive control. (b) of Fig. 15 is a graph showing overall scores of respective mice in the case of using the ointment 2. (c) is a graph showing overall scores of respective mice in the case of using the negative control. "A" through "I" in (a) through (c) of Fig. 15 indicate respective mice used in the test. In (a) through (c) of Fig. 15, "1" through "5" along the horizontal axis indicate the start of the treatment (day 0), day 7 after the start of the treatment, day 14 after the start of the treatment, day 21 after the start of the treatment, and day 28 after the start of the treatment, respectively.

As shown in (a) of Fig. 15, on the points in time of 1, 2, 3, 4, and 5, the mouse A had a score of 0, 2, 4.5, 7.5, and 5.5 respectively, the mouse B had a score of 0, 4, 7.5, 7.5, and 5, respectively, and the mouse C had a score of 0, 2.5, 7.5, 7.5, and 4, respectively. As shown in (b) of Fig. 15, on the points in time of 1, 2, 3, 4, and 5, the mouse D had a score of 0, 3, 8, 8.5, and 7.5, respectively, the mouse E had a score of 0, 4.5, 5.5, 5.5, and 4.5, respectively, and the mouse F had a score of 0, 2, 6, 5, and 5, respectively. As shown in (c) of Fig. 15, on the points in time of 1, 2, 3, 4, and 5, the mouse G had a score of 0, 4, 5, 6, and 7.5, respectively, the mouse H had a score of 0, 5.5, 7, 9, and 8.5, respectively, and the mouse I had a score of 0, 3.5, 6.5, 7, and 8, respectively.

Fig. 16 is a graph showing an average value of the scores shown in (a) of Fig. 15 (a cross), an average value of the scores shown in (b) of Fig. 15 (a regular square), and an average value of the scores shown in (c) of Fig. 5 (a diamond). As shown in Fig. 16, on the points in time of 1, 2, 3, 4, and 5, the mice for which the ointment 2 was used had a score of 0, 3.16, 6.5, 6.3, and 5.66, respectively, the mice for which the positive control was used had a score of 0, 2.83, 6.5, 7.5, and 4.83, respectively, and the mice for which the control was used had a score of 0, 4.33, 6.16, 7.33, and 8, respectively.

As shown in Figs. 15 and 16, a score increased as the time passed in the case of using the negative control, whereas a score decreased as the time passed in the case of using the ointment 2 and in the case of using the positive control. This indicates that atopic dermatitis is not treated by application of the negative control but is treated by application of the ointment 2 or the positive control. Since the score obtained in the case of using the ointment 2 and the score obtained in the case of using the positive control are equivalent values, the ointment 2 and the positive control appears to have equivalent effects on atopic dermatitis.

Fig. 17 shows photographs ((a) through (d)) of the backs of the mice, photographs ((e) through (h)) of the slices of skin subjected to the hematoxylin-eosin staining, and photograph ((i) through (1)) of the slices of skin subjected to the toluidine blue staining. In Fig. 17, (a), (e) and (i) are photographs of untreated mice, to which neither the ointment containing the antigen of mite nor the above-described external medicines were applied, (b), (f) and (j) are photographs of the mice to which the ointment containing the antigen of mite and the control were applied, (c), (g) and (k) are photographs of the mice to which the ointment containing the antigen of mite and the ointment 2 were applied, and (d), (h) and (l) are photographs of the mice to which the ointment containing the antigen of mite and the positive control were applied. In (e) through (h) of Fig. 17, epidermis is indicated by an arrow, and a cell of the dermis is indicated by an arrowhead. In (i) through (l) of Fig. 17, a mast cell is indicated by an arrow.

Comparison of (a) through (d) of Fig. 17 shows that erythema, edema, and erosion are developed (atopic dermatitis is developed) in the mice to which the ointment containing the antigen of mite was applied. In the mice to which the ointment 2 was applied and the mice to which the positive control was applied, degrees of erythema, edema, and erosion are reduced as compared with the mice to which the negative control was applied.

Comparison of (e) through (h) of Fig. 17 shows that, in the mice to which the negative control was applied, noticeable infiltration of a cell into dermis is observed as compared with the untreated mice. On the other hand, the mice to which the ointment 2 was applied and the mice to which the positive control was applied has less infiltration of a cell into epidermis as compared with the mice to which the negative control was applied.

Comparison of (i) through (l) of Fig. 17 shows that infiltration of a mast cell is observed in the mice to which the negative control was applied, which infiltration is not observed in the untreated mice. On the other hand, infiltration of a mast cell as observed in the mice to which the negative control was applied is observed less in the mice to which the ointment 2 was applied and the mice to which the positive control was applied.

Based on the results, it was found that application of the ointment 2 alleviates atopic dermatitis. In the mice to which the ointment 2 was applied and the mice to which the positive control was applied, a symptom of atopic dermatitis was alleviated equally.

### [Treatment of erythema of tuberous sclerosis complex patients with use of an external medicine of the present invention]

Erythema is a site in which angiogenesis is actively carried out. Erythema is known to be often developed in a patient affected by a disease (tuberous sclerosis complex or the like) caused by activation of mTOR. The inventors considered that a mechanism of the development of erythema in such a disease was as follows.

That is, as a result of activation of mTOR in a cell of the patient, an activity of HIF1α is increased, so that activated HIF1α forms a heterodimer with HIF1β. The heterodimer binds to HRE sequence of DNA so as to induce transcription of VEGF, so that expression of VEGF is increased. Expressed VEGF causes angiogenesis. In this manner, erythema is developed.

The inventors thus considered that, since activation of mTOR is involved in development of erythema, an external medicine of the present invention was effective for treating erythema. In view of this, the following test was conducted in the present example to find out that (i) VEGF was expressed in a cell of a patient with tuberous sclerosis complex and (ii) sirolimus inhibits the expression of VEGF. It was confirmed that the external medicine of the present invention had an effect on erythema of the patient with tuberous sclerosis complex.

### <Confirmation of VEGF expression caused by angiofibroma isolated from a patient with tuberous sclerosis complex>

### (Test method)

A cell of severe angiofibroma, a cell of moderate angiofibroma, and a cell of mild angiofibroma were isolated from a patient having severe angiofibroma, a patient having moderate angiofibroma, and a patient having mild angiofibroma, respectively. The cells of angiofibroma were isolated from the faces of the respective patients in accordance with a general primary culturing method of a fibroblast (see "New Cultured Cell Experiment Methods for Molecular Biology Study (Bunshi Seibutsugaku Kenkyu no Tame no Shin Baiyo Saibo Jikken Ho*)",* separate volume of Experimental Medicine, Biomanual UP Series, second revised edition, edited by Toshio Kuroki et al., Yodosha). The cells obtained from the patients were used with the patients' consent.

Each of the isolated cells was cultured for 48 hours in a DME culture medium at a temperature of 37°C in a 5% CO₂ environment. After that, a culture supernatant was collected. Then, a concentration of VEGF in the culture supernatant was measured with use of a kit (Quantikine^{®} Human VEGF manufactured by R&D SYSTEMS^{®}) for detecting VEGF.

### (Test results)

Results of the test are shown in Fig. 18. In Fig. 18, A indicates a cell of severe angiofibroma, B indicates a cell of moderate angiofibroma, and C indicates a cell of mild angiofibroma. As shown in Fig. 18, a concentration of VEGF was 468.857 pg/mL in a case of using the cell of severe angiofibroma, 260.286 pg/mL in a case of using the cell of moderate angiofibroma, and 147.429 pg/mL in a case of using the cell of mild angiofibroma. It was thus found that (i) VEGF was expressed in the cells of angiofibroma and (ii) an expression level of VEGF increased as a symptom of angiofibroma became severer.

### <Confirmation of inhibition of VEGF expression by sirolimus>

### (Test method)

A cell of angiofibroma, a human fibroblast, and HaCat, each of which was isolated from a patient with tuberous sclerosis complex, were used. Isolation of the cell of angiofibroma was carried out by removing angiofibroma from a patient who wished the angiofibroma to be removed and then using the primary culturing method. The human fibroblast was obtained from a tissue of a patient, who did not have tuberous sclerosis complex (TSC), with use of the primary culturing method. HaCat was purchased from German Cancer Research Center (dkfz). The cells obtained from the respective patients were used with the patients' consent.

The cell of angiofibroma, the human fibroblast, and HaCat were each cultured with use of a DME culture medium at a temperature of 37°C in a 5% CO₂ environment. During the culture of these cells, sirolimus was added to each of the culture mediums so as to have a final concentration of 0 nM (that is, no sirolimus is added to a culture medium (a control)), 1 nM, 10 nM, or 20 nM. The cells were each incubated again at a temperature of 37°C in a 5% CO₂ environment, and a culture supernatant was collected 48 or 72 hours after sirolimus was added. A concentration of VEGF in the collected culture supernatant was measured with use of a kit (Quantikine^{®} Human VEGF manufactured by R&D SYSTEMS^{®}) for detecting VEGF.

### (Test results)

Results of the test are shown in Fig. 19.

(a) of Fig. 19 is a graph showing a concentration (pg/mL) of VEGF in a culture supernatant 48 hours after sirolimus was added during the culture of the cell of angiofibroma. (b) of Fig. 19 is a graph showing (i) a concentration (pg/mL) of VEGF in a culture supernatant 72 hours after sirolimus was added during the culture of HaCat, the human fibroblast (normal fibro), and the cell of angiofibroma (TSC AF fibro) and (ii) a concentration (pg/mL) of VEGF in a culture supernatant after similarly carrying out 72 hours of culture without adding sirolimus during the culture of these cells. (c) of Fig. 19 is a graph showing a rate of decrease (ratio: a concentration of VEGF after treatment with 20 nM of sirolimus / a concentration of VEGF before the treatment with 20 nM of sirolimus) in concentration of VEGF, which decrease was caused by treating each of the three types of cells with sirolimus. (d) of Fig. 19 is a graph showing a concentration (pg/mL) of VEGF in a culture supernatant 48 hours after sirolimus was added during the culture of HaCat.

As shown in (a) of Fig. 19, a concentration of VEGF was 260.2857 pg/mL in a case where the cell of angiofibroma was not treated with sirolimus (0 nM), a concentration of VEGF was 113.1428 pg/mL in a case where the cell of angiofibroma was treated with 1 nM of sirolimus, and a concentration of VEGF was 92.4285 pg/mL in a case where the cell of angiofibroma was treated with 10 nM of sirolimus.

According to (a) of Fig. 19, it was shown that an expression level of VEGF decreased as a concentration of sirolimus was increased.

(b) of Fig. 19, a concentration of VEGF was 1849.9 pg/mL in a case where HaCat was not treated with sirolimus (a control); a concentration of VEGF was 749.9 pg/mL in a case where HaCat was treated with 20 nM of sirolimus, a concentration of VEGF was 1492.7 pg/mL in a case where the human fibroblast was not treated with sirolimus (a control); a concentration of VEGF was 501.3 pg/mL in a case where the human fibroblast was treated with 20 nM of sirolimus; a concentration of VEGF was 858.4 pg/mL in a case where the cell of angiofibroma was not treated with sirolimus (a control); and a concentration of VEGF was 168 pg/mL in a case where the cell of angiofibroma was treated with 20 nM of sirolimus.

(c) of Fig. 19 is a graph showing a result obtained in a case where a concentration, shown in (b) of Fig. 19, of VEGF corresponding to a case where a cell was treated with 20 nM of sirolimus is divided by a concentration, shown in (b) of Fig. 19, of VEGF corresponding to a case where the cell was not treated with sirolimus. As shown in (c) of Fig. 19, in the case of HaCat, a concentration of VEGF became 0.4053 pg/mL (decreased to about 40%) by treatment with 20 nM of sirolimus; in the case of the human fibroblast, a concentration of VEGF became 0.3358 pg/mL (decreased to about 30%) by treatment with 20 nM of sirolimus; and in the case of the cell of angiofibroma, a concentration of VEGF became 0.1957 pg/mL (decreased to about 20%) by treatment with 20 nM of sirolimus.

According to (d) of Fig. 19, it was shown that an expression level of VEGF was decreased as a concentration of sirolimus was increased.

According to (b) and (c) of Fig. 19, it was found that the cell of angiofibroma was more apt to be affected by inhibition of VEGF expression by sirolimus as compared with the human fibroblast and HaCat.

Further, how an expression level of HIF1α changes in a case of treating melanocytes with various concentrations of sirolimus was examined. Specifically, melanocytes were cultured at a temperature of 37°C in 5% a CO₂ environment. During culture of these cells, sirolimus was added to a culture medium so as to have a final concentration of 0 nM (that is, no sirolimus was added to the culture medium (a control)), 1 nM, or 10 nM. The cells were incubated again at a temperature of 37°C in a 5% CO₂ environment, and melanocytes were collected 48 hours after sirolimus was added. An amount of mRNA of HIF1α expressed in melanocytes was measured by real time PCR, and the amount of mRNA was corrected on the basis of an amount of mRNA of GAPDH.

Results of the measurement are shown in Fig. 29. As is clear from Fig. 29, an expression level of HIF1α decreased in melanocytes treated with sirolimus.

### <Confirmation of an effect of an external medicine of the present invention on erythema>

To erythema of a patient with tuberous sclerosis complex, the ointment 3 was applied twice a day for 12 weeks. Fig. 20 shows a photograph (a) of erythema taken before the start of the application of the ointment 3 and a photograph (b) of erythema taken 12 weeks after the start of the application of the ointment 3. An arrow in Fig. 20 indicates erythema. The erythema shown in (a) of Fig. 20 has disappeared in (b) of Fig. 20. It was thus confirmed that erythema could be treated by application of the ointment 3.

Thus, use of the ointment 3 actually caused an affected part of human, in which part angiogenesis is actively carried out, to disappear almost completely. The external medicine of the present invention is therefore also effective against rosacea in which angiogenesis is likewise actively carried out.

### [Condition of sirolimus in ointment]

In order to check what condition sirolimus is in in the ointments prepared in the present example, the condition of propylene carbonate, which is a solvent of sirolimus, in the ointments was examined.

Propylene carbonate was colored into blue. Next, colored propylene carbonate was added to a mixture of liquid paraffin, solid paraffin, and white petrolatum, which are components of the ointments prepared in the present example. Further, colored propylene carbonate was added to white petrolatum to serve as a control. In a case where colored propylene carbonate was added to the mixture, propylene carbonate precipitated in the form of fine particles as shown in a reagent bottle on the left of (a) of Fig. 21. In a case where colored propylene carbonate was added to white petrolatum, propylene carbonate precipitated without forming fine particles as shown in a reagent bottle on the right of (b) of Fig. 21.

Next, the mixture in which propylene carbonate had precipitated was stirred with use of Planetary Centrifugal Mixer at normal temperature at 2,000 rpm for 1 minute, then at 1,000 rpm for 5 minutes, and then at 500 rpm for 3 minutes. The same operation was carried out with respect to white petrolatum in which colored propylene carbonate precipitated. Results of stirring are shown in (b) of Fig. 21. In (b) of Fig. 21, the reagent bottle on the left shows a result obtained by stirring the mixture in which colored propylene carbonate precipitated, and the reagent bottle on the right shows a result obtained by stirring white petrolatum in which colored propylene carbonate precipitated. As shown in (b) of Fig. 21, it is recognizable with naked eyes that propylene carbonate has a larger particle size in a case where propylene carbonate is stirred together with white petrolatum than in a case where propylene carbonate is stirred together with the mixture.

Further, an ointment prepared by stirring propylene carbonate together with the mixture, and an ointment prepared by stirring propylene carbonate together with white Petrolatum, were observed with use of a microscope. Results of the observation are shown in Fig. 22. (a) of Fig. 22 is a microphotograph of the ointment prepared with use of the mixture, and (c) of Fig. 22 is an enlarged view of (a) of Fig. 22. (b) of Fig. 22 is a microphotograph of the ointment prepared with use of white petrolatum, and (d) of Fig. 22 is an enlarged view of (b) of Fig. 22. A bar in (c) and (d) of Fig. 22 indicates 25 µm. In Fig. 22, a particle of propylene carbonate is indicated by an arrow, and water (black) is indicated by an arrowhead.

As is clear from (a) and (c) of Fig. 22, in the ointment prepared with use of the mixture, propylene carbonate is dispersed in the form of uniform fine particles each having a size of 10 µm or less. On the other hand, as is clear from (b) and (d) of Fig. 22, in the ointment prepared with use of white petrolatum, propylene carbonate is dispersed in the form of nonuniform fine particles of various sizes.

Such a result is likely to be obtained not only in the case of using propylene carbonate alone, but also in a case of using propylene carbonate in which sirolimus is dissolved. That is, sirolimus is likely to be dispersed as uniform fine particles in each of the ointments prepared in the present example. Since sirolimus is thus dispersed as uniform fine particles, application of an ointment prepared in the present example to skin is likely to (i) significantly increase the absorption of sirolimus into an affected part through skin and (ii) cause sirolimus to stay in the affected part without leaking into blood.

### [Study concerning bases]

### <Preparation Example 7: Preparation of a gel containing 0.2% by mass of sirolimus>

2 mg of the reagent of sirolimus (produced by Calbiochem; 553210) was dissolved in 145 mg of isopropanol (produced by Wako Pure Chemical Industries, Ltd.; 166-04836). 147 mg of a melt thus obtained was mixed with a mixture of 16 mg of Carbopol^{®} 934NF Polymer (produced by Lubrizol Corporation) and 833 mg of H₂O. With a resultant mixture, 4 mg of tris(hydroxymethyl)aminomethane (produced by Wako Pure Chemical Industries, Ltd.; 203-06272) was mixed. In this way, a gel (gel 4) containing 0.2% by mass of sirolimus was prepared.

### <Preparation Example 8: Preparation of a gel containing 0.2% by mass of sirolimus>

A gel (gel 5) containing 0.2% by mass of sirolimus was prepared by carrying out the same operation as in Preparation Example 7 except that Rapamune was used instead of the reagent of sirolimus. Specifically, 2 mg of a tablet of Rapamune was pulverized and sieved through a 75-µm mesh in order to remove impurities (a vehicle etc.), and a ground product thus obtained was dissolved in 145 mg of isopropanol.

### <Preparation Example 9: Preparation of an ointment containing 0.2% by mass of sirolimus>

2 mg of the reagent of sirolimus (produced by Calbiochem; 553210) was dissolved in 58 mg of propylene carbonate (produced by Wako Pure Chemical Industries, Ltd.; 168-04972). Then, 45 mg of solid paraffin (produced by Wako Pure Chemical Industries, Ltd.; 415-25791) and 895 mg of white petrolatum (propet) (produced by Maruishi Pharmaceutical Co. Ltd, Merck & Co., Inc., or Astra Japan) were mixed together at 70°C to be dissolved.

A mixture thus obtained was cooled down to 40°C, and then the above-described melt of the reagent of sirolimus and propylene carbonate was added in the mixture. A resultant mixture was stirred with use of Planetary Centrifugal Mixer (manufactured by THINKY CORPORATION; Nano Pulverizer NP-100) at normal temperature at 2,000 rpm for 1 minute, then at 1,000 rpm for 5 minutes, and then at 500 rpm for 3 minutes. Thus prepared was an ointment (ointment 7) containing 0.2% by mass of sirolimus.

### <Preparation Example 10: Preparation of an ointment containing 0.2% by mass of sirolimus>

2 mg of the reagent of sirolimus (produced by Calbiochem; 553210) was dissolved in 50 mg of propylene carbonate (produced by Wako Pure Chemical Industries, Ltd.; 168-04972). Then, 10 mg of liquid paraffin (produced by Wako Pure Chemical Industries, Ltd.; 128-04375), 35 mg of solid paraffin (produced by Wako Pure Chemical Industries, Ltd.; 415-25791), and 895 mg of white petrolatum (propet) (produced by Maruishi Pharmaceutical Co. Ltd, Merck & Co., Inc., or Astra Japan) were mixed together at 70°C to be dissolved.

A mixture thus obtained was cooled down to 40°C, and then the above-described melt of the reagent of sirolimus and propylene carbonate was added. A resultant mixture was stirred with use of Planetary Centrifugal Mixer (manufactured by THINKY CORPORATION; Nano Pulverizer NP-100) at normal temperature at 2,000 rpm for 1 minute, then at 1,000 rpm for 5 minutes, and then at 500 rpm for 3 minutes. Thus prepared was an ointment (ointment 8) containing 0.2% by mass of sirolimus.

### <Checking the amounts of external medicines of the present invention absorbed by artificial skin - 2>

### (Test method and test results - 1)

The external medicines of the present invention prepared with use of Rapamune were examined as to how a composition of a base affects the amount of an external medicine absorbed by artificial skin. Specifically, with respect to the ointment 3, the ointment 7, the gel 3, and the gel 5, the amount of each external medicine absorbed via artificial skin was measured.

Descriptions on how the measurement was carried out are omitted here since they are already described in the section [Checking the amounts of external medicines of the present invention absorbed via artificial skin]. Descriptions are given only on test results. Note that in the present example, 3 samples of each of the ointment 3, the ointment 7, the gel 3, and the gel 5 were tested, each sample being in an amount of 40 mg.

Results of the test are shown in Fig. 30, and numerical data actually obtained in the test are shown in Table 2.

**[Table 2]**

| | Ointment 3 | Ointment 7 | Gel 3 | Gel 5 |
|---|---|---|---|---|
| Sample 1 | 0.34 | 0.23 | 1.14 | 0.19 |
| Sample 2 | 0.29 | 0.44 | 0.85 | 0.47 |
| Sample 3 | 0.15 | 0.17 | 1.23 | 0.16 |
| mean | 0.26 | 0.28 | 1.073333333 | 0.273333333 |
| SD | 0.098488578 | 0.141774469 | 0.19857828 | 0.170977581 |

As is clear from Fig. 30 and Table 2, in the case of the external medicines of the present invention prepared with use of Rapamune, the gel 3 had the largest amount of sirolimus absorbed by artificial skin among the ointment 3, the ointment 7, the gel 3, and the gel 5.

### (Test method and test results - 2)

The external medicines of the present invention prepared with use of the reagent of sirolimus were examined as to how a composition of a base affects the amount of an external medicine absorbed via artificial skin. Specifically, with respect to the ointment 2, the ointment 8, the gel 2, and the gel 4, the amount of each external medicine absorbed via artificial skin was measured.

Descriptions on how the measurement was carried out are omitted here since they are already described in the section [Checking the amounts of external medicines of the present invention absorbed via artificial skin]. Descriptions are given only on test results. Note that in the present example, 3 samples of each of the ointment 2, the ointment 8, the gel 2, and the gel 4 were tested, each sample being in an amount of 40 mg.

Results of the test are shown in Fig. 31, and numerical data actually obtained in the test are shown in Table 3.

**[Table 3]**

| | Ointment 2 | Ointment 8 | Gel 2 | Gel 4 |
|---|---|---|---|---|
| Sample 1 | 1.03 | 1.17 | 1.98 | 2.78 |
| Sample 2 | 1.17 | 1.04 | 1.69 | 1.14 |
| Sample 3 | 1.06 | 0.73 | 1.8 | 1.14 |
| mean | 1.086666667 | 0.98 | 1.5775 | 1.686666667 |
| SD | 0.073711148 | 0.226053091 | 0.50598913 | 0.946854441 |

As is clear from Fig. 31 and Table 3, in the case of the external medicines of the present invention prepared with use of the reagent of sirolimus, the gel 2 had the largest amount of sirolimus absorbed by artificial skin among the ointment 2, the ointment 8, the gel 2, and the gel 4.

### (Test method and test results - 3)

The test results of the gels 2 through 5 described in the section (Test method and test results - 1) and the section (Test method and test results - 2) are collectively shown in a graph of Fig. 32.

As is clear from Fig. 32, comparison between (i) the external medicines of the present invention prepared with use of the reagent of sirolimus and (ii) the external medicines of the present invention prepared with use of Rapamune shows that the external medicines of the present invention prepared with use of the reagent of sirolimus had a greater amount of sirolimus absorbed via artificial skin.

### [Study of effect on skin]

Gels and ointments containing various concentrations of sirolimus (0% by mass, 0.05% by mass, 0.2% by mass, and 0.4% by mass) were each applied once, in an amount of 100 mg, to both ears of a BALB/c mouse. After 1 hour, 8 hours, 24 hours, 48 hours, and 7 days, whether or not inflammation was caused was checked. Further, the gels and the ointments were each applied to the back of a BALB/c mouse once a day for 5 consecutive days to check whether or not local dermatitis was caused by the external application.

With the gels and the ointments, no abnormality was observed in skin in any time in the duration.

### Industrial Applicability

The present invention can topically treat a skin disease. Accordingly, the present invention is applicable not only to the pharmaceutical industry as described in the present invention but also to the cosmetics industry.

## Claims

1. An external medicine for topically treating a skin disease, comprising a gel composition or an ointment composition, each of which contains sirolimus and/or a derivative thereof.

2. The external medicine as set forth in claim 1, wherein:
the gel composition is a gelated solution containing sirolimus and/or a derivative thereof.

3. The external medicine as set forth in claim 2, wherein:
the solution is a dissolving solution in which sirolimus and/or a derivative thereof is dissolved in isopropanol and/or ethanol.

4. The external medicine as set forth in any one of claims 1 through 3, wherein:
the skin disease is at least one disease selected from the group consisting of skin tumor, atopic dermatitis, rosacea, keloid, pigmented macule, hypopigmented macule, abnormal vascular malformation in skin, benign vascular tumor in skin, and epithelial nevus.

5. The external medicine as set forth in claim 4, wherein:
the skin tumor is at least one tumor selected from the group consisting of skin tumor of tuberous sclerosis complex, seborrheic keratosis, and skin tumor of Recklinghausen's disease.

6. The external medicine as set forth in claim 4, wherein:
the pigmented macule is brownish pigmented macule.

7. The as set forth in claim 6, wherein:
the brownish pigmented macule is nevus spilus or café-au-lait spots.

8. The external medicine as set forth in claim 4, wherein:
the hypopigmented macule is vitiligo.

9. The external medicine as set forth in any one of claims 1 through 8, wherein:
the gel composition contains at least one of Carbopol^{®} 934NF, water, isopropanol, and tris(hydroxymethyl)aminomethane.

10. The external medicine as set forth in claim 9, wherein:
a mass ratio of (i) sirolimus and/or a derivative thereof, (ii) Carbopol^{®} 934NF, (iii) water, (iv) isopropanol, and (iv) tris(hydroxymethyl)aminomethane is 2-4 : 16 : 490-833 : 145-488 : 4; and
a sum of (i) the mass ratio of sirolimus and/or the derivative thereof, (ii) the mass ratio of water, and (iii) the mass ratio of isopropanol is 980.

11. The external medicine as set forth in any one of claims 1 through 8, wherein:
the ointment composition contains at least one of propylene carbonate, solid paraffin, white petrolatum, liquid paraffin, and white beeswax.

12. The external medicine as set forth in claim 11, wherein:
a mass ratio of (i) sirolimus and/or a derivative thereof, (ii) propylene carbonate, (iii) solid paraffin, (iv) white petrolatum, (v) liquid paraffin, and (vi) the white beeswax is 0.3-10 : 50-59.4 : 30-45 : 895 : 0-10 : 0-5; and
a sum of (i) the mass ratio of sirolimus and/or the derivative thereof, (ii) the mass ratio of propylene carbonate, (iii) the mass ratio of solid paraffin, (iv) the mass ratio of liquid paraffin, and (v) the mass ratio of the white beeswax is 105.

13. A composition for preparing an external medicine recited in any one of claims 1 through 12, the composition comprising sirolimus and/or a derivative thereof.

14. A kit for preparing an external medicine recited in any one of claims 1 through 12, the kit comprising sirolimus and/or a derivative thereof.

15. A method for producing an external medicine recited in any one of claims 1 through 12, the method comprising the step of preparing a gel composition or an ointment composition, each of which contains sirolimus and/or a derivative thereof.
